# EUROPEAN PATENT SPECIFICATION

(11) **EP 3 122 707 B1**
(45) Date of publication and mention of the grant of the patent: **25.04.2018**
(21) Application number: 15716307.2
(22) Date of filing: 27.03.2015
(51) Int. Cl.: C07B 37/04, C07C 67/343, C07C 41/30, C07C 1/32, C07C 45/70, C07C 211/45, C07C 231/12, C07C 233/00, C07D 263/54, C07C 253/30, C07D 213/127, C07D 213/16

(54) **METHOD FOR COUPLING A FIRST AROMATIC COMPOUND TO A SECOND AROMATIC COMPOUND**
VERFAHREN ZU KOPPLUNG EINER ERSTEN AROMATISCHEN VERBINDUNG MIT EINER ZWEITEN AROMATISCHEN VERBINDUNG
PROCÉDÉ DE COUPLAGE D'UN PREMIER COMPOSÉ AROMATIQUE À UN SECOND COMPOSÉ AROMATIQUE

(30) Priority: 28.03.2014 US 201461971671 P; 16.03.2015 US 201562133615 P
(43) Date of publication of application: 01.02.2017
(73) Proprietor: Dow Global Technologies LLC, Midland, MI 48674 (US); Dow AgroSciences LLC, Indianapolis, IN 46268 (US)
(72) Inventor: HANLEY, Patrick S., Midland, MI 48674 (US); WHITEKER, Gregory T., Indianapolis, IN 46268 (US); OBER, Matthias S., Midland, MI 48674 (US); KRUPER, William J. Jr., Sanford, MI 48657 (US); KRASOVSKIY, Arkady L., Midland, MI 48674 (US)
(74) Representative: Houghton, Mark Phillip
(86) International application number: PCT/US2015/023008
(87) International publication number: WO 2015/148931

(56) References cited:
- EP-A1- 1 127 861
- WO-A1-2014/031791
- GREGORY P ROTH ET AL: "Palladium cross-coupling reactions of aryl fluorosulfonates: An alternative to triflate chemistry", THE JOURNAL OF ORGANIC CHEMISTRY, AMERICAN CHEMICAL SOCIETY, US, vol. 56, no. 11, 1 January 1991 (1991-01-01), pages 3493-3496, XP002682972, ISSN: 0022-3263

## Description

### Background

Suzuki-Miyaura coupling is a valuable synthetic method for coupling a first aromatic compound to a second aromatic compound, thereby forming a new carbon-carbon bond between the aromatic compounds. In one common Suzuki reaction the first aromatic compound is substituted by a halide. In some instances, the first aromatic compound used in the Suzuki coupling is prepared from an aromatic compound having a hydroxyl substituent. In one common Suzuki reaction the second aromatic compound includes a boron-containing substituent.

It is known that triflates, having the formula F₃CSO₂-, may be used in the place of the halides in Suzuki couplings, however the expense of triflic anhydride (CF₃SO₂)₂O has limited the use of triflates in Suzuki couplings to the production of fine chemicals. Further, the atom economy of triflic anhydride is low since half of the molecule is expended as monomeric triflate anion (CF₃SO₂⁻) following functionalization of a phenolic precursor. In some instances Suzuki coupling reactions involving triflates exhibit sensitivity to water under basic conditions.

It is also known that aryl methanesulfonates are suitable for aryl-aryl cross-coupling reactions. One drawback of aryl-aryl cross-couping using aryl methanesulfonates is that these reactions require expensive palladium catalysts. Another drawback of aryl-aryl cross-coupling using aryl methanesulfonates is low atom economy.

US 2001/0020104 discloses a process for preparing substituted benzyl compounds and toluene derivatives.

WO 2014/031791 discloses methods of preparing aromatic compounds.

Gregory P. Roth et al., in The Journal of Organic Chemistry, American Chemical Society, vol. 56, no. 11, 1 Jan 1991, pages 3493-3496, disclose "Palladium cross-coupling reactions of aryl fluorosulfonates: An alternative to triflate chemistry".

When performing a Suzuki coupling using either a triflate or methanesulfonate, it is common to perform the reaction in two steps, a first step comprising replacing the hydroxyl group on the first aromatic compound with the triflate or the methanesulfonate, and a second step comprising coupling the first aromatic compound with the second aromatic compound. A separation step is generally required between the first and second steps.

It would be desirable to have a replacement for triflates and methanesulfonates for the Suzuki coupling.

### Statement of Invention

The present invention, in all its aspects is set out in the accompanying claims.

In one aspect, there is provided a particular method of coupling a first aromatic compound having a fluorosulfonate substituent to a second aromatic compound having a boron-containing substituent.

In one aspect, there is provided a particular method of coupling a first aromatic compound having a hydroxyl substituent to a second aromatic compound having a boron-containing substituent in a one-pot reaction.

### Detailed Description

Unless otherwise indicated, numeric ranges, for instance "from 2 to 10," are inclusive of the numbers defining the range (e.g., 2 and 10).

Unless otherwise indicated, ratios, percentages, parts, and the like are by weight.

As used herein, unless otherwise indicated, the phrase "molecular weight" refers to the number average molecular weight as measured in conventional manner.

"Alkyl," as used in this specification, whether alone or as part of another group (e.g., in dialkylamino), encompasses straight and branched chain aliphatic groups having the indicated number of carbon atoms. If no number is indicated (e.g., aryl-alkyl-), then 1-12 alkyl carbons are contemplated. Preferred alkyl groups include, without limitation, methyl, ethyl, propyl, isopropyl, butyl, isobutyl, sec-butyl, tert-butyl, pentyl, hexyl and tert-octyl.

The term "heteroalkyl" refers to an alkyl group as defined above with one or more heteroatoms (nitrogen, oxygen, sulfur, phosphorus) replacing one or more carbon atoms within the radical, for example, an ether or a thioether.

An "aryl" group refers to any functional group or substituent derived from an aromatic ring. In one instance, aryl refers to an aromatic moiety comprising one or more aromatic rings. In one instance, the aryl group is a C₆-C₁₈ aryl group. In one instance, the aryl group is a C₆-C₁₀ aryl group. In one instance, the aryl group is a C₁₀-C₁₈ aryl group. Aryl groups contain 4n+2 pi electrons, where n is an integer. The aryl ring may be fused or otherwise attached to one or more heteroaryl rings, aromatic or non-aromatic hydrocarbon rings or heterocycloalkyl rings. Preferred aryls include, without limitation, phenyl, naphthyl, anthracenyl, and fluorenyl. Unless otherwise indicated, the aryl group is optionally substituted with 1 or more substituents that are compatible with the syntheses described herein. Such substituents include, but are not limited to, sulfonate groups, boron-containing groups, alkyl groups, nitro groups, halogens, cyano groups, carboxylic acids, esters, amides, C₂-C₈ alkene, and other aromatic groups. Other substituents are known in the art. Unless otherwise indicated, the foregoing substituent groups are not themselves further substituted.

"Heteroaryl" refers to any functional group or substituent derived from an aromatic ring and containing at least one heteroatom selected from nitrogen, oxygen, and sulfur. Preferably, the heteroaryl group is a five or six-membered ring. The heteroaryl ring may be fused or otherwise attached to one or more heteroaryl rings, aromatic or non-aromatic hydrocarbon rings or heterocycloalkyl rings. Examples of heteroaryl groups include, without limitation, pyridine, pyrimidine, pyridazine, pyrrole, triazine, imidazole, triazole, furan, thiophene, oxazole, thiazole. The heteroaryl group may be optionally substituted with one or more substituents that are compatible with the syntheses described herein. Such substituents include, but are not limited to, fluorosulfonate groups, boron-containing groups, C₁-C₈ alkyl groups, nitro groups, halogens, cyano groups, carboxylic acids, esters, amides, C₂-C₈ alkene and other aromatic groups. Other substituents are known in the art. Unless otherwise indicated, the foregoing substituent groups are not themselves further substituted.

"Aromatic compound" refers to a ring system having 4n+2 pi electrons where n is an integer.

As noted above, the present disclosure describes a process for coupling a first aromatic compound to a second aromatic compound. This process is shown generally in Equation 1, whereby a first aromatic compound having a hydroxyl group is first reacted with SO₂F₂ and a base and is second reacted with a second aromatic compound having a boron-containing substituent in the presence of a catalyst. It is understood that where a hydroxyl group is indicated, the hydroxyl group could be deprotonated to form a phenolate (e.g. the deprotonation step could be performed prior to introduction of A¹ to the reaction mixture or after the introduction to the reaction mixture).

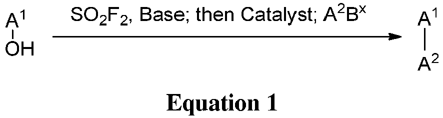

Unexpectedly, it has been found that the reaction of Equation 1 may be performed as a one-pot reaction, as compared to performing the reaction indiscrete steps. Without being limited by theory, it is anticipated that the reaction shown in Equation 1 proceeds along the same reaction path whether performed as a one-pot reaction or as discrete steps. When performed in discrete steps, the first step comprises reacting a first aromatic compound having a hydroxyl substituent with SO₂F₂ to yield the product shown in Equation 2, and the second step comprises reacting the product of Equation 2 with a second aromatic compound having a boron-containing substituent to yield the product shown in Equation 3.

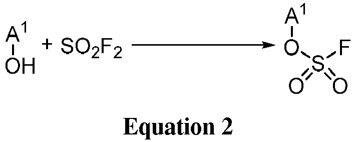

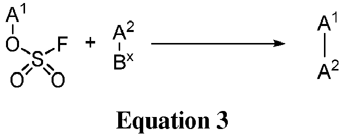

In one instance, the process involves a one-pot reaction where a first aromatic compound having a hydroxyl group is first reacted with SO₂F₂ and a base and is second reacted with a second aromatic compound having a boron-containing substituent in the presence of a catalyst, as shown generally in Equation 1. Without being limited by theory, it is expected that Equation 3 is the same general reaction as depicted by step 2) of the reaction shown in Equation 1.

As used in Equation 1, Equation 2 and Equation 3, the first aromatic compound is identified as A¹ and the second aromatic compound is identified as A². The first aromatic compound is either an aryl group or a heteroaryl group. The second aromatic compound is either an aryl group or a heteroaryl group. The result of the reactions shown in Equation 3 and Equation 3 is the formation of a new carbon-carbon bond between the first aromatic compound and the second aromatic compound, thereby coupling the first aromatic compound to the second aromatic compound.

As noted above in the first step of Equation 3 and in Equation 2, the first aromatic compound is bonded to a fluorosulfonate group. A fluorosulfonate group refers to O-fluorosulfonate of the formula -OSO₂F. O-fluorosulfonate may be synthesized from sulfuryl fluoride. The fluorosulfonate group serves as a leaving group from the first aromatic compound. Without being limited by theory, the sulfuryl atom of the fluorosulfonate group is bonded to the oxygen of the hydroxyl group of the first aromatic compound.

As noted above, the second aromatic compound includes a boron-containing substituent as identified in Equation 1 and Equation 3 as B^{x}. In one instance, the boron-containing substituent is of the formula -BF₃⁻M⁺ where M⁺ is an alkali metal cation or an unsubstituted ammonium ion. In one instance, the boron of the boron-containing substituent has one or more alkyl substituent, for example, 9-borabicyclo[3.3.1]nonane. In another instance, the boron-containing substituent is of the formula shown in Equation 4.

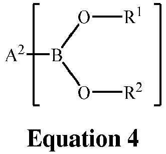

In Equation 4 R¹ and R² are each independently C₁₋₁₈ alkyl, C₃₋₁₈ cycloalkyl, C₆₋₁₈ aryl, or H. In another instance, R¹ and R² are covalently linked to each other to form a ring that includes -R¹-O-B-O-R²-. In one instance one or more of R¹ and R² are boron, for example, in a boroxane. Boron-containing substituents which are known to be suitable for typical Suzuki reactions are suitable here.

As noted above in Equation 1 and Equation 3, the first aromatic compound is reacted with the second aromatic compound in a reaction mixture. The reaction mixture includes a catalyst having at least one group 10 atom. In some instances, the reaction mixture also includes a ligand, and a base. The group 10 atoms include nickel, palladium and platinum.

The catalyst is provided in a form suitable to the reaction conditions. In one instance, the catalyst is provided on a substrate. In one instance, the catalyst having at least one group 10 atom is generated *in situ* from one or more precatalysts and one or more ligands. Examples of palladium precatalysts include, but are not limited to, Palladium(II) acetate, Palladium(II) chloride, Dichlorobis(acetonitrile)palladium(II), Dichlorobis(benzonitrile)palladium(II), Allylpalladium chloride dimer, Palladium(II) acetylacetonate, Palladium(II) bromideBis(dibenzylideneacetone)palladium(0), Bis(2-methylallyl)palladium chloride dimer, Crotylpalladium chloride dimer, Dichloro(1,5-cyclooctadiene)palladium(II), Dichloro(norbornadiene)palladium(II), Palladium(II) trifluoroacetate, Palladium(II) benzoate, Palladium(II) trimethylacetate, Palladium(II) oxide, Palladium(II) cyanide, Tris(dibenzylideneacetone)dipalladium(0), Palladium(II) hexafluoroacetylacetonate, cis-Dichloro(N,N,N',N'-tetramethylethylenediamine) palladium(II), and Cyclopentadienyl[(1,2,3-n)-1-phenyl-2-propenyl]palladium(II).

In one instance, nickel-based catalysts are used. In another instance, platinum-based catalysts are used. In yet another instance, a catalyst including one or more of nickel, platinum and palladium -based catalysts are used.

In one instance, pyridine-enhanced precatalyst preparation stabilization and initiation (PEPPSI) type catalysts are used, for example, [1,3-Bis(2,6-Diisopropylphenyl)imidazol-2-ylidene](3-chloropyridyl)palladium(II) dichloride, and (1,3-Bis(2,6-diisopropylphenyl) imidazolidene) (3-chloropyridyl) palladium(II) dichloride.

Examples of nickel precatalysts include, but are not limited to, nickel(II) acetate, nickel(II) chloride, Bis(triphenylphosphine)nickel(II) dichloride, Bis(tricyclohexylphosphine) nickel(II) dichloride, [1,1'-Bis(diphenylphosphino)ferrocene]dichloronickel(II), Dichloro[1,2-bis(diethylphosphino)ethane]nickel(II), Chloro(1-naphthyl)bis(triphenylphosphine)nickel(II), 1,3-Bis(2,6-diisopropylphenyl)imidazolium chloride, Bis(1,5-cyclooctadiene)nickel(0), Nickel(II) chloride ethylene glycol dimethyl ether complex, [1,3-Bis(diphenylphosphino) propane]dichloronickel(II), [1,2-Bis(diphenylphosphino)ethane]dichloronickel(II), and Bis(tricyclohexylphosphine)nickel(0).

The ligand used in the reaction mixture is preferably selected to generate the selected catalyst from a pre-catalyst. For example, the ligand may be a phosphine ligand, a carbene ligand, an amine-based ligand, a carboxylate based ligand, an aminodextran, an aminophosphine-based ligands or an N-heterocyclic carbene-based ligand. In one instance, the ligand is monodentate. In one instance, the ligand is bidentate. In one instance, the ligand is polydentate.

Suitable phosphine ligands may include, but are not limited to, mono- and bi-dentate phosphines containing functionalized aryl or alkyl substituents or their salts. For example, suitable phosphine ligands include, but are not limited to, triphenylphosphine; Tri(*o-*tolyl)phosphine; Tris(4-methoxyphenyl)phosphine; Tris(pentafluorophenyl)phosphine; Tri(*p-*tolyl)phosphine; Tri(2-furyl)phosphine; Tris(4-chlorophenyl)phosphine; Di(1-adamantyl)(1-naphthoyl)phosphine; Benzyldiphenylphosphine; 1,1'-Bis(di-t-butylphosphino)ferrocene; (-)-1,2-Bis((2R,5R)-2,5-dimethylphospholano)benzene; (-)-2,3-Bis[(2R,5R)-2,5-dimethylphospholanyl]-1-[3,5-bis(trifluoromethyl)phenyl]-1H-pyrrole-2,5-dione; 1,2-Bis(diphenylphosphino)benzene; 2,2'-Bis(diphenylphosphino)-1,1'-binaphthyl; 2,2'-Bis(diphenylphosphino)-1,1'-biphenyl, 1,4-Bis(diphenylphosphino)butane; 1,2-Bis(diphenylphosphino)ethane; 2-[Bis(diphenylphosphino)methyl]pyridine; 1,5-Bis(diphenylphosphino)pentane; 1,3-Bis(diphenylphosphino)propane; 1,1'-Bis(di-i-propylphosphino)ferrocene; (S)-(-)-5,5'-Bis[di(3,5-xylyl)phosphino]-4,4'-bi-1,3-benzodioxole; tricyclohexylphosphine(referred to herein as PCy3); Tricyclohexylphosphine tetrafluoroborate (referred to herein as PCy3•HBF₄); N-[2-(di-1-adamantylphosphino) phenyl]morpholine; 2-(Di-t-butylphosphino)biphenyl; 2-(Di-t-butylphosphino)-3,6-dimethoxy-2',4',6'-tri-i-propyl-1,1'-biphenyl; 2-Di-t-butylphosphino-2'-(N,N-dimethylamino) biphenyl; 2-Di-t-butylphosphino-2'-methylbiphenyl; Dicyclohexylphenylphosphine; 2-(Dicyclohexylphosphino)-3,6-dimethoxy-2',4',6'-tri-i-propyl-1; 2-(Dicyclohexylphosphino)-2'-(N,N-dimethylamino)biphenyl; 2-Dicyclohexylphosphino-2',6'-dimethylamino-1,1'-biphenyl; 2-Dicyclohexylphosphino-2',6'-di-i-propoxy-1,1'-biphenyl; 2-Dicyclohexylphosphino-2'-methylbiphenyl; 2-[2-(Dicyclohexylphosphino)phenyl]-1-methyl-1H-indole; 2-(Dicyclohexylphosphino)-2',4',6'-tri-i-propyl-1,1'-biphenyl; [4-(N,N-Dimethylamino)phenyl]di-t-butylphosphine; 9,9-Dimethyl-4,5-bis(diphenylphosphino) xanthene; (R)-(-)-1-[(S)-2-(Diphenylphosphino)ferrocenyl]ethyldicyclohexylphosphine; Tribenzylphosphine; Tri-t-butylphosphine; Tri-n-butylphosphine; and 1,1'-Bis(diphenylphosphino)ferrocene.

Suitable amine and aminophosphine-based ligands include any combination of monodentate or bidentate alkyl and aromatic amines including, but not limited to, pyridine, 2,2'-Bipyridyl, 4,4'-Dimethyl-2,2'-dipyridyl, 1,10-Phenanthroline, 3,4,7,8-Tetramethyl-1,10-phenanthroline, 4,7-Dimethoxy-1,10-phenanthroline, *N,N,N',N'*-Tetramethylethylenediamine, 1,3-Diaminopropane, ammonia, 4-(Aminomethyl)pyridine, (1*R*,2*S*,9*S*) -(+)-11-Methyl-7,11-diazatricyclo[7.3.1.0^{2,7}]tridecane, 2,6-Di-*tert*-butylpyridine, 2,2'-Bis[(4*S*)-4-benzyl-2-oxazoline], 2,2-Bis((4*S*)-(-)-4-isopropyloxazoline)propane, 2,2'-Methylenebis[(4*S*)-4-phenyl-2-oxazoline], and 4,4'-di-tert-butyl-2,2'bipyridyl. In addition, aminophosphine ligands such as 2-(Diphenylphosphino)ethylamine, 2-(2-(Diphenylphosphino)ethyl)pyridine, (1R,2R)-2-(diphenylphosphino)cyclohexanamine, an aminodextran and 2-(Di-*tert-*butylphosphino)ethylamine.

Suitable carbene ligands include N-heterocyclic carbene (NHC) based ligands, including, but not limited to, 1,3-Bis(2,4,6-trimethylphenyl)imidazolinium chloride, 1,3-Bis(2,6-diisopropylphenyl)imidazolium chloride, 1,3-Bis-(2,6-diisopropylphenyl) imidazolinium chloride, 1,3-Diisopropylimidazolium chloride, and 1,3-Dicyclohexylbenzimidazolium chloride.

The base used in the reaction mixture is selected to be compatible with the catalyst, the boron-containing substituent and the fluorosulfonate. Suitable bases include, but are not limited to, carbonate salts, phosphate salts, acetate salts and carboxylic acid salts. Unexpectedly, it has been found that inorganic bases are suitable in the reaction mixture.

Examples of carbonate salts include, but are not limited to, lithium carbonate, sodium carbonate, potassium carbonate, rubidium carbonate, cesium carbonate, ammonium carbonate, substituted ammonium carbonates, and the corresponding hydrogen carbonate salts. Examples of phosphate salts include, but are not limited to, lithium phosphate, sodium phosphate, potassium phosphate, rubidium phosphate, cesium phosphate, ammonium phosphate, substituted ammonium phosphates, and the corresponding hydrogen phosphate salts.Examples of acetate salts include, but are not limited to, lithium acetate, sodium acetate, potassium acetate, rubidium acetate, cesium acetate, ammonium acetate, and substituted ammonium acetates.

Other bases include, but are not limited to, salts of formate, fluoroacetate, and propionate anions with lithium, sodium, potassium, rubidium, cesium, ammonium, and substituted ammonium cations; metal hydroxides, such as lithium hydroxide, sodium hydroxide, potassium hydroxide, metal dihydroxides such as magnesium dihydroxide, calcium dihydroxide, strontium dihydroxide, and barium dihydroxide; metal trihydroxides such as aluminum trihydroxide, gallium trihydroxide, indium trihydroxide, thallium trihydroxide; non nucleophilic organic amines such as triethylamine, N,N-diisopropylethylamine, 1,4-diazabicyclo[2.2.2]octane (DABCO), 1,5-Diazabicyclo[4.3.0] non-5-ene (DBN), 1,8-Diazabicyclo[5.4.0]undec-7-ene (DBU); bis(silyl)amide salts such as the lithium, sodium, and potassium salts of bis(trimethylsilyl)amide; alkoxide salts such as the lithium, sodium, and potassium salts of t butoxide; and 1,8-bis(dimethylamino) naphthalene; metal fluorides, such as sodium fluoride, potassium fluoride, cesium fluoride, silver fluoride, tetra butyl ammonium fluoride, ammonium fluoride, triethyl ammonium fluoride.

Examples of amine bases, such as alkylamines and heteroarenes include, but are not limited to, triethylamine, pyridine, morpholine, 2,6-lutidine, triethylamine, N,N-Dicyclohexylmethylamine, and diisopropylamine.

In one instance, the base is used in the presence of a phase-transfer catalyst. In another instance, the base is used in the presence of water. In yet another instance, the base is used in the presence of an organic solvent. In still another instance, the base is used in the presence of one or more of a phase-transfer catalyst, water or an organic solvent.

Preferably, at least one equivalent of base is present for each equivalent of fluorosulfonate. In some embodiments, no more than 10 equivalents of base are present for each equivalent of fluorosulfonate. In some embodiments, at least 2 equivalents of base are present for each equivalent of fluorosulfonate. In some embodiments, no more than 6 equivalents of base are present for each equivalent of fluorosulfonate.

The solvent in the reaction mixture is selected such that it is suitable for use with the reactants, the catalyst, the ligand and the base. For example, suitable solvents include toluene, xylenes (*ortho*-xylene, *meta*-xylene, *para*-xylene or mixtures thereof), benzene, water, methanol, ethanol, 1-propanol, 2-propanol, *n*-butanol, 2-butanol, pentanol, hexanol, *tert*-butyl alcohol, *tert*-amyl alcohol, ethylene glycol, 1,2-propanedioal, 1,3-propanediol, glycerol, *N*-methyl-2-pyrrolidone, acetonitrile, *N,N*-dimethylformamide, methyl acetate, ethyl acetate, propyl acetate, isopropyl acetate, triacetin, acetone, methyl ethyl ketone, and ethereal solvents, such as 1,4-dioxane, tetrahydrofuran, 2-methyltetrahydrofuran, diethylether, cyclopenyl methyl ether, 2-butyl ethyl ether, dimethoxyethane, polyethyleneglycol. In one instance, the solvent includes any combination of the solvents described herein, in, or in the absence of, a surfactant. In one instance, the sulfuryl fluoride is used neat at a sufficiently low temperature that the sulfuryl fluoride is in a liquid.

In one instance, water is included in the reaction mixture. One benefit of using fluorosulfonates as compared to triflates, is that the reaction can be carried out without a subsequent separation step, or with a simple separation step. In Suzuki couplings involving triflates, a dedicated purification step is required to remove byproducts since the products and the byproducts typically occupy the same phase. In the reaction schemes described herein, the byproducts are either in the gas phase, and will bubble out spontaneously or with a simple degassing step, or will partition into the aqueous phase, which is easily separable. As such, the reaction scheme described herein provides additional benefits as compared to Suzuki couplings involving triflates.

In one instance, the reaction described herein is completed as a one-pot reaction as shown in Equation 1. In a first step, an aromatic compound having an alcohol substituent is added to a reaction mixture in the presence of sulfuryl fluoride and a base. The base may be any of the bases described herein, including, without limitation, amine bases and inorganic bases. This first step couples the fluorosulfonate substituent to the oxygen of the hydroxyl group. To the reaction mixture formed during this first step is added a second aromatic compound having a boron-containing substituent and a catalyst. The catalyst may be a suitable group 10 catalyst, including, without limitation, platinum, palladium and nickel catalysts. The product of this second step is a bi-aryl compound formed by coupling the first aromatic compound and the second aromatic compound.

Some embodiments of the invention will now be described in detail in the following Examples. Unless stated otherwise, reported yields are ± 5%.

### Example 1. p-tolyl sulfofluoridate, phenylboronic acid reacted to yield 4-phenyltoluene

In this Example, p-tolyl sulfofluoridate is reacted with phenylboronic acid to yield 4-phenyltoluene as shown in Equation 5.

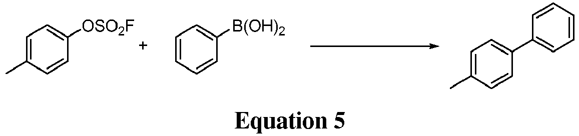

In a N₂ filled glovebox, twenty four 20 mL scintillation vials are each charged with the reactants listed in Table 1. To the reactants is added a palladium precatalyst (listed in Table 2), a ligand (listed in Table 3), a base (listed in Table 4) and a solvent (listed in Table 5). 0.75mL of water is also added to a portion of the vials. Table 6 lists the Palladium precatalyst, ligand, base and solvent which are added to each vial. Table 6 also lists whether water is added to each vial.

**Table 1**

| Reactant | Equivalents | Amount |
|---|---|---|
| p-tolyl sulfofluoridate | 1 | 0.190 g |
| phenylboronic acid | 2 | 0.244 g |

**Table 2**

| Palladium Precatalyst | Equivalents | Amount |
|---|---|---|
| palladium acetate (referred to herein as Pd(OAc)₂) | 0.02 | 0.0045 g |
| cyclopentadienyl[(1,2,3-η)-1-phenyl-2-propenyl]palladium(II) (referred to herein as CpPd(cinnamyl)) | 0.02 | 0.0058 g |

**Table 3**

| Ligand | Equivalents | Amount |
|---|---|---|
| Triphenylphosphine (referred to herein as PPh₃) | 0.05 | 0.013 g |
| 1,3-bis(diphenylphosphino)propane (referred to herein as DPPP) | 0.05 | 0.021 g |
| 1,1'-bis(diphenylphosphino)ferrocene (referred to herein as DPPF) | 0.05 | 0.028 g |
| 2-dicyclohexylphosphino-2',4',6'-triisopropylbiphenyl (referred to herein as XPhos) | 0.05 | 0.024 g |
| 2-[2-(dicyclohexylphosphino)phenyl]-1-methyl-1H-indole (referred to herein as CMPhos) | 0.05 | 0.020 g |

**Table 4**

| Base | Equivalents | Amount |
|---|---|---|
| potassium phosphate (referred to herein as K₃PO₄) | 2 | 0.425 g |
| triethylamine (referred to herein as NEt₃) | 2 | 0.28 mL |

**Table 5**

| Solvent | Equivalents | Amount |
|---|---|---|
| tert-Butyl alcohol | - | 2.32 g |
| 1,4-dioxane | - | 3.0 mL |
| toluene | - | 3.0 mL |

**Table 6**

| Vial | Pd Source | Ligand | Base | Solvent | Water | Yield |
|---|---|---|---|---|---|---|
| 1 | CpPd(cinnamyl) | PPh₃ | NEt₃ | 1,4-dioxane | yes | 75% |
| 2 | CpPd(cinnamyl) | DPPF | NEt₃ | toluene | no | 25% |
| 3 | CpPd(cinnamyl) | DPPP | K₃PO₄ | toluene | no | 4% |
| 4 | Pd(OAc)₂ | DPPP | NEt₃ | 1,4-dioxane | no | 5% |
| 5 | Pd(OAc)₂ | DPPP | NEt₃ | toluene | yes | 9% |
| 6 | CpPd(cinnamyl) | CMPhos | K₃PO₄ | toluene | no | 55% |
| 7 | Pd(OAc)₂ | PPh₃ | K₃PO₄ | 1,4-dioxane | no | 48% |
| 8 | CpPd(cinnamyl) | DPPF | K₃PO₄ | tert-Butyl alcohol | no | 50% |
| 9 | Pd(OAc)₂ | XPhos | NEt₃ | toluene | no | 96% |
| 10 | Pd(OAc)₂ | CMPhos | NEt₃ | toluene | yes | 99% |
| 11 | CpPd(cinnamyl) | PPh₃ | K₃PO₄ | tert-Butyl alcohol | yes | 86% |
| 12 | Pd(OAc)₂ | PPh₃ | NEt₃ | tert-Butyl alcohol | no | 86% |
| 13 | CpPd(cinnamyl) | XPhos | K₃PO₄ | tert-Butyl alcohol | no | 33% |
| 14 | CpPd(cinnamyl) | DPPP | K₃PO₄ | 1,4-dioxane | yes | 10% |
| 15 | Pd(OAc)₂ | PPh₃ | K₃PO₄ | 1,4-dioxane | no | 37% |
| 16 | Pd(OAc)₂ | DPPP | K₃PO₄ | tert-Butyl alcohol | no | 20% |
| 17 | Pd(OAc)₂ | XPhos | NEt₃ | tert-Butyl alcohol | yes | 93% |
| 18 | Pd(OAc)₂ | CMPhos | NEt₃ | toluene | yes | 106% |
| 19 | Pd(OAc)₂ | DPPF | K₃PO₄ | toluene | yes | 77% |
| 20 | CpPd(cinnamyl) | XPhos | K₃PO₄ | toluene | yes | 96% |
| 21 | CpPd(cinnamyl) | CMPhos | NEt₃ | 1,4-dioxane | no | 93% |
| 22 | Pd(OAc)₂ | DPPF | NEt₃ | tert-Butyl alcohol | yes | 45% |
| 23 | Pd(OAc)₂ | PPh₃ | NEt₃ | tert-Butyl alcohol | no | 86% |
| 24 | Pd(OAc)₂ | CMPhos | K₃PO₄ | 1,4-dioxane | yes | 100% |

The reaction mixture in each vial is heated to 80°C for 17 hours with vigorous stirring. Following heating, the reaction mixture in each vial is cooled to room temperature, and thereafter 5 mL of trimethoxybenzene in ethyl acetate (1.33 M solution) is added to the reaction mixture in each vial and each vial is shaken. An aliquot from the organic layer of the reaction mixture of each vial is analyzed by gas chromatography, and the yield of 4-phenyltoluene is determined from the integration of the 4-phenyltoluene peak compared to that of the integration of the trimethoxybenzene peak, with a correction factor of 1.55. The yield of 4-phenyltoluene for each vial is listed in Table 6.

### Example 2. p-tolyl sulfofluoridate, phenyl boronic acid reacted to yield 4-phenyltoluene

In this Example, p-tolyl sulfofluoridate is reacted with phenylboronic acid to yield 4-phenyltoluene as shown in Equation 5.

In a nitrogen-filled glovebox, two 20 mL scintillation vials ("vial A" and "vial B") are each charged with 190 mg of 1.00 mmol p-tolylsulfurofluoridate, 183 mg of 1.50 mmol phenyl boronic acid, 1.8 mL of 1,4-dioxane and 0.5 mL of water.

A third 20 mL scintillation vial ("vial C") is charged with 22.5 mg of 0.1 mmol palladium acetate, 80.7 mg of 0.2 mmol CMPhos, and 1 mL of 1,4-dioxane. 279 µL of 2.0 mmol triethylamine and 200 µL of the solution of vial C are pippeted to vial A.

A fourth 20 mL scintillation vial ("vial D") is charged with 22.5 mg of 0.1 mmol palladium acetate, 52.5 mg of 0.2 mmol PPh₃ and 1 mL of 1,4-dioxane. 279 µL of 2.0 mmol triethylamine and 200 µL of the solution of vial D is pippeted to vial B.

Vials A and B are stirred at 80 °C for 17 hours. To each of vials A and B, 1 mL of brine and 2 mL of ethyl acetate are added. The organic layers of each vial are separated via pipette. The aqueous layer of each vial is extracted several times with ethyl acetate. The remaining organic layers of each vial are extracted and combined with the respective previously-separated organic layer. Each organic layer is brought to a total weight of 10.00 g by the addition of ethyl acetate.

A 1.00 g aliquot is removed from each organic layer for quantitative gas chromatography / mass spectroscopy analysis

The two reactions are individually worked up by addition of 1 mL brine and 2 mL ethyl acetate and separation of the organic layer via pipette. The aqueous layer is extracted several times with ethyl acetate and the combined organic phases are each brought to a total weight of exactly 10.00 g by addition of ethyl acetate.

From each of these 10.00 g organic phase samples, a 1.00 g aliquot is removed. To each 1.00 g aliquot is added 150 µL of 1,3,5-trimethoxybenzene in ethyl acetate (0.133 M solution, 0.02 mmol, 0.2 eq) as a standard solution. These standard solutions are analyzed using gas chromatography and mass spectroscopy.

Samples of pure starting material (0.1 mmol) and pure product (0.1 mmol) in 1.00 g ethyl acetate plus 150 µL of the standard solution are prepared in order to determine response factors required for quantification.

The remaining 9.00 g organic phase of the CMPhos catalyzed reaction is concentrated and purified by automated flash chromatography (dry loading onto silica loading cartridge, 24g Grace normal phase silica purification cartridge, isocratic hexanes). The product elutes as a single peak and is isolated by evaporation of the solvent followed by drying under high vacuum.

For the reaction preformed in vial C (use of CMPhos as the ligand), the yield of 4-phenyltoluene, as calculated by gas chromatography, is 93%. The yield of 4-phenyltoluene, as calculated by isolation, was 79%.

For the reaction performed in vial D (use of PPh₃ as the ligand), the yield of 4-phenyltoluene, as calculated by gas chromatography, was 82%.

### Example 3. p-tolyl sulfofluoridate, tolyl boronic acid reacted to yield 4,4'-dimethyl-1,1'-biphenyl

In this Example, p-tolyl sulfofluoridate is reacted with tolyl boronic acid to yield 4,4'-dimethyl-1,1'-biphenyl as shown in Equation 6.

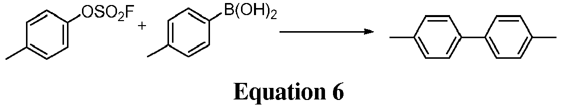

In a nitrogen-filled glovebox, two 20 mL scintillation vials ("vial A" and "vial B") are each charged with 190 mg of 1.00 mmol p-tolylsulfurofluoridate, 204 mg of 1.50 mmol tolyl boronic acid, 2.8 mL of 1,4-dioxane and 0.5 mL of water.

A third 20 mL scintillation vial ("vial C") is charged with 22.5 mg of 0.1 mmol palladium acetate, 80.7 mg of 0.2 mmol CMPhos, and 1 mL of 1,4-dioxane. 279 µL of 2.0 mmol triethylamine and 200 µL of the solution of vial C are pippeted to vial A.

A fourth 20 mL scintillation vial ("vial D") is charged with 22.5 mg of 0.1 mmol palladium acetate, 52.5 mg of 0.2 mmol PPh₃ and 1 mL of 1,4-dioxane. 279 µL of 2.0 mmol triethylamine and 200 µL of the solution of vial D is pippeted to vial B.

Vials A and B are stirred at 80 °C for 17 hours. To each of vials A and B, 1 mL of brine and 2 mL of ethyl acetate are added. The organic layers of each vial are separated via pipette. The aqueous layer of each vial is extracted several times with ethyl acetate. The remaining organic layers of each vial are extracted and combined with the respective previously-separated organic layer. Each organic layer is brought to a total weight of 10.00 g by the addition of ethyl acetate.

A 1.00 g aliquot is removed from each organic layer for quantitative gas chromatography / mass spectroscopy analysis

The two reactions are individually worked up by addition of 1 mL brine and 2 mL ethyl acetate and separation of the organic layer via pipette. The aqueous layer is extracted several times with ethyl acetate and the combined organic phases are each brought to a total weight of exactly 10.00 g by addition of ethyl acetate.

From each of these 10.00 g organic phase samples, a 1.00 g aliquot is removed. To each 1.00 g aliquot is added 150 µL of 1,3,5-trimethoxybenzene in ethyl acetate (0.133 M solution, 0.02 mmol, 0.2 eq) as a standard solution. These standard solutions are analyzed using gas chromatography and mass spectroscopy.

Samples of pure starting material (0.1 mmol) and pure product (0.1 mmol) in 1.00 g ethyl acetate plus 150 µL of the standard solution are prepared in order to determine response factors required for quantification.

The remaining 9.00 g organic phase of the CMPhos catalyzed reaction is concentrated and purified by automated flash chromatography (dry loading onto silica loading cartridge, 24g Grace normal phase silica purification cartridge, isocratic hexanes). The product elutes as a single peak and is isolated by evaporation of the solvent followed by drying under high vacuum.

For the reaction preformed in vial C (use of CMPhos as the ligand), the yield of 4,4'-dimethyl-1,1'-biphenyl, as calculated by gas chromatography, is 95%. The yield of 4,4'-dimethyl-1,1'-biphenyl, as calculated by isolation, is 99%.

For the reaction performed in vial D (use of PPh₃ as the ligand), the yield of 4,4'-dimethyl-1,1'-biphenyl, as calculated by gas chromatography, is 88%.

### 4-(trifluoromethyl)phenylsulfofluoridate, phenyl boronic acid reacted to yield 4-trifluoromethyl-1,1'-biphenyl

In this Example, 4-(trifluoromethyl)phenylsulfofluoridate is reacted with phenylboronic acid to yield 4-trifluoromethyl-1,1'-biphenyl as shown in Equation 7.

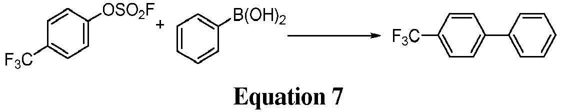

In a nitrogen-filled glovebox, two 20 mL scintillation vials ("vial A" and "vial B") are each charged with 244 mg of 1.00 mmol 4-(trifluoromethyl)phenylsulfofluoridate, 183 mg of 1.50 mmol phenyl boronic acid, 1.8 mL of 1,4-dioxane and 0.5 mL of water.

A third 20 mL scintillation vial ("vial C") is charged with 22.5 mg of 0.1 mmol palladium acetate, 80.7 mg of 0.2 mmol CMPhos, and 1 mL of 1,4-dioxane. 279 µL of 2.0 mmol triethylamine and 200 µL of the solution of vial C are pippeted to vial A.

A fourth 20 mL scintillation vial ("vial D") is charged with 22.5 mg of 0.1 mmol palladium acetate, 52.5 mg of 0.2 mmol PPh₃ and 1 mL of 1,4-dioxane. 279 µL of 2.0 mmol triethylamine and 200 µL of the solution of vial D is pippeted to vial B.

Vials A and B are stirred at 80 °C for 17 hours. To each of vials A and B, 1 mL of brine and 2 mL of ethyl acetate are added. The organic layers of each vial are separated via pipette. The aqueous layer of each vial is extracted several times with ethyl acetate. The remaining organic layers of each vial is extracted and combined with the respective previously-separated organic layer. Each organic layer is brought to a total weight of 10.00 g by the addition of ethyl acetate.

A 1.00 g aliquot is removed from each organic layer for quantitative gas chromatography / mass spectroscopy analysis

The two reactions are individually worked up by addition of 1 mL brine and 2 mL ethyl acetate and separation of the organic layer via pipette. The aqueous layer is extracted several times with ethyl acetate and the combined organic phases are each brought to a total weight of exactly 10.00 g by addition of ethyl acetate.

From each of these 10.00 g organic phase samples, a 1.00 g aliquot is removed. To each 1.00 g aliquot is added 150 µL of 1,3,5-trimethoxybenzene in ethyl acetate (0.133 M solution, 0.02 mmol, 0.2 eq) as a standard solution. These standard solutions are analyzed using gas chromatography and mass spectroscopy.

Samples of pure starting material (0.1 mmol) and pure product (0.1 mmol) in 1.00 g ethyl acetate plus 150 µL of the standard solution are prepared in order to determine response factors required for quantification.

The remaining 9.00 g organic phase of the CMPhos catalyzed reaction is concentrated and purified by automated flash chromatography (dry loading onto silica loading cartridge, 24g Grace normal phase silica purification cartridge, isocratic hexanes). The product elutes as a single peak and is isolated by evaporation of the solvent followed by drying under high vacuum.

For the reaction preformed in vial C (use of CMPhos as the ligand), the yield of 4-trifluoromethyl-1,1'-biphenyl, as calculated by gas chromatography, is 97%. The yield of 4-trifluoromethyl-1,1'-biphenyl, as calculated by isolation, is 99%.

For the reaction performed in vial D (use of PPh₃ as the ligand), the yield of 4-trifluoromethyl-1,1'-biphenyl, as calculated by gas chromatography, is 104%.

### Example 5. 4-(trifluoromethyl)phenylsulfofluoridate, tolyl boronic acid reacted to yield 4-phenyltoluene

In this Example, 4-(trifluoromethyl)phenylsulfofluoridate is reacted with tolyl boronic acid to yield 4-phenyltoluene as shown in Equation 8.

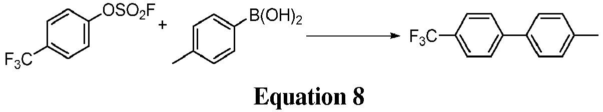

In a nitrogen-filled glovebox, two 20 mL scintillation vials ("vial A" and "vial B") are each charged with 244 mg of 1.00 mmol 4-(trifluoromethyl)phenylsulfofluoridate, 204 mg of 1.50 mmol tolyl boronic acid, 2.8 mL of 1,4-dioxane and 0.5 mL of water.

A third 20 mL scintillation vial ("vial C") is charged with 22.5 mg of 0.1 mmol palladium acetate, 80.7 mg of 0.2 mmol CMPhos, and 1 mL of 1,4-dioxane. 279 µL of 2.0 mmol triethylamine and 200 µL of the solution of vial C are pippeted to vial A.

A fourth 20 mL scintillation vial ("vial D") is charged with 22.5 mg of 0.1 mmol palladium acetate, 52.5 mg of 0.2 mmol PPh₃ and 1 mL of 1,4-dioxane. 279 µL of 2.0 mmol triethylamine and 200 µL of the solution of vial D is pippeted to vial B.

Vials A and B are stirred at 80 °C for 17 hours. To each of vials A and B, 1 mL of brine and 2 mL of ethyl acetate are added. The organic layers of each vial are separated via pipette. The aqueous layer of each vial is extracted several times with ethyl acetate. The remaining organic layers of each vial are extracted and combined with the respective previously-separated organic layer. Each organic layer is brought to a total weight of 10.00 g by the addition of ethyl acetate.

A 1.00 g aliquot is removed from each organic layer for quantitative gas chromatography / mass spectroscopy analysis

The two reactions are individually worked up by addition of 1 mL brine and 2 mL ethyl acetate and separation of the organic layer via pipette. The aqueous layer is extracted several times with ethyl acetate and the combined organic phases are each brought to a total weight of exactly 10.00 g by addition of ethyl acetate.

From each of these 10.00 g organic phase samples, a 1.00 g aliquot is removed. To each 1.00 g aliquot is added 150 µL of 1,3,5-trimethoxybenzene in ethyl acetate (0.133 M solution, 0.02 mmol, 0.2 eq) as a standard solution. These standard solutions are analyzed using gas chromatography and mass spectroscopy.

Samples of pure starting material (0.1 mmol) and pure product (0.1 mmol) in 1.00 g ethyl acetate plus 150 µL of the standard solution are prepared in order to determine response factors required for quantification.

The remaining 9.00 g organic phase of the CMPhos catalyzed reaction is concentrated and purified by automated flash chromatography (dry loading onto silica loading cartridge, 24g Grace normal phase silica purification cartridge, isocratic hexanes). The product elutes as a single peak and is isolated by evaporation of the solvent followed by drying under high vacuum.

For the reaction preformed in vial C (use of CMPhos as the ligand), the yield of 4-phenyltoluene, as calculated by gas chromatography, is 99%. The yield of 4-phenyltoluene, as calculated by isolation, is 90%.

For the reaction performed in vial D (use of PPh₃ as the ligand), the yield of 4-phenyltoluene, as calculated by gas chromatography, is 101 %.

### Example 6. pyridin-3-yl sulfofluoridate, phenyl boronic acid reacted to yield 3-phenylpyridine

In this Example, pyridin-3-yl sulfofluoridate is reacted with phenylboronic acid to yield 3-phenylpyridine as shown in Equation 9.

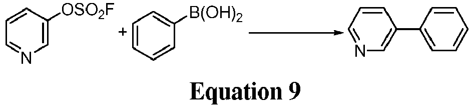

In a nitrogen-filled glovebox, two 20 mL scintillation vials ("vial A" and "vial B") are each charged with 177 mg of 1.00 mmol pyridin-3-yl sulfofluoridate, 183 mg of 1.50 mmol phenyl boronic acid, 1.8 mL of 1,4-dioxane and 0.5 mL of water.

A third 20 mL scintillation vial ("vial C") is charged with 22.5 mg of 0.1 mmol palladium acetate, 80.7 mg of 0.2 mmol CMPhos, and 1 mL of 1,4-dioxane. 279 µL of 2.0 mmol triethylamine and 200 µL of the solution of vial C are pippeted to vial A.

A fourth 20 mL scintillation vial ("vial D") is charged with 22.5 mg of 0.1 mmol palladium acetate, 52.5 mg of 0.2 mmol PPh₃ and 1 mL of 1,4-dioxane. 279 µL of 2.0 mmol triethylamine and 200 µL of the solution of vial D is pippeted to vial B.

Vials A and B are stirred at 80 °C for 17 hours. To each of vials A and B, 1 mL of brine and 2 mL of ethyl acetate are added. The organic layers of each vial are separated via pipette. The aqueous layer of each vial is extracted several times with ethyl acetate. The remaining organic layers of each vial are extracted and combined with the respective previously-separated organic layer. Each organic layer was brought to a total weight of 10.00 g by the addition of ethyl acetate.

A 1.00 g aliquot is removed from each organic layer for quantitative gas chromatography / mass spectroscopy analysis

The two reactions are individually worked up by addition of 1 mL brine and 2 mL ethyl acetate and separation of the organic layer via pipette. The aqueous layer is extracted several times with ethyl acetate and the combined organic phases are each brought to a total weight of exactly 10.00 g by addition of ethyl acetate.

From each of these 10.00 g organic phase samples, a 1.00 g aliquot is removed. To each 1.00 g aliquot is added 150 µL of 1,3,5-trimethoxybenzene in ethyl acetate (0.133 M solution, 0.02 mmol, 0.2 eq) as a standard solution. These standard solutions are analyzed using gas chromatography and mass spectroscopy.

Samples of pure starting material (0.1 mmol) and pure product (0.1 mmol) in 1.00 g ethyl acetate plus 150 µL of the standard solution are prepared in order to determine response factors required for quantification.

The remaining 9.00 g organic phase of the CMPhos catalyzed reaction is concentrated and purified by automated flash chromatography (dry loading onto silica loading cartridge, 24g Grace normal phase silica purification cartridge, isocratic hexanes). The product elutes as a single peak and is isolated by evaporation of the solvent followed by drying under high vacuum.

For the reaction preformed in vial C (use of CMPhos as the ligand), the yield of 3-(p-tolyl)pyridine, as calculated by gas chromatography, is 45%.

For the reaction performed in vial D (use of PPh₃ as the ligand), the yield of 3-phenylpyridine, as calculated by gas chromatography, is 91 %. The yield of 3-(p-tolyl)pyridine, as calculated by isolation, is 68%.

### pyridin-3-yl sulfofluoridate, p-tolyl boronic acid reacted to yield 3-(p-tolyl)pyridine

In this Example, pyridin-3-yl sulfurofluoridate is reacted with p-tolyl boronic acid to yield 3-(p-tolyl)pyridine as shown in Equation 10.

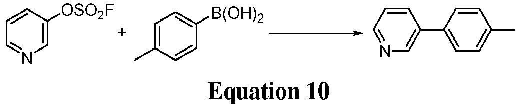

In a nitrogen-filled glovebox, two 20 mL scintillation vials ("vial A" and "vial B") are each charged with 177 mg of 1.00 mmol pyridin-3-yl sulfofluoridate, 204 mg of 1.50 mmol p-tolyl boronic acid, 1.8 mL of 1,4-dioxane and 0.5 mL of water.

A third 20 mL scintillation vial ("vial C") is charged with 22.5 mg of 0.1 mmol palladium acetate, 80.7 mg of 0.2 mmol CMPhos, and 1 mL of 1,4-dioxane. 279 µL of 2.0 mmol triethylamine and 200 µL of the solution of vial C are pippeted to vial A.

A fourth 20 mL scintillation vial ("vial D") is charged with 22.5 mg of 0.1 mmol palladium acetate, 52.5 mg of 0.2 mmol PPh₃ and 1 mL of 1,4-dioxane. 279 µL of 2.0 mmol triethylamine and 200 µL of the solution of vial D is pippeted to vial B.

Vials A and B are stirred at 80 °C for 17 hours. To each of vials A and B, 1 mL of brine and 2 mL of ethyl acetate are added. The organic layers of each vial are separated via pipette. The aqueous layer of each vial is extracted several times with ethyl acetate. The remaining organic layers of each vial are extracted and combined with the respective previously-separated organic layer. Each organic layer is brought to a total weight of 10.00 g by the addition of ethyl acetate.

A 1.00 g aliquot is removed from each organic layer for quantitative gas chromatography / mass spectroscopy analysis

The two reactions are individually worked up by addition of 1 mL brine and 2 mL ethyl acetate and separation of the organic layer via pipette. The aqueous layer is extracted several times with ethyl acetate and the combined organic phases are each brought to a total weight of exactly 10.00 g by addition of ethyl acetate.

From each of these 10.00 g organic phase samples, a 1.00 g aliquot is removed. To each 1.00 g aliquot is added 150 µL of 1,3,5-trimethoxybenzene in ethyl acetate (0.133 M solution, 0.02 mmol, 0.2 eq) as a standard solution. These standard solutions are analyzed using gas chromatography and mass spectroscopy.

Samples of pure starting material (0.1 mmol) and pure product (0.1 mmol) in 1.00 g ethyl acetate plus 150 µL of the standard solution are prepared in order to determine response factors required for quantification.

The remaining 9.00 g organic phase of the CMPhos catalyzed reaction is concentrated and purified by automated flash chromatography (dry loading onto silica loading cartridge, 24g Grace normal phase silica purification cartridge, isocratic hexanes). The product elutes as a single peak and is isolated by evaporation of the solvent followed by drying under high vacuum.

For the reaction preformed in vial C (use of CMPhos as the ligand), the yield of 3-(p-tolyl)pyridine, as calculated by gas chromatography, is 59%.
For the reaction performed in vial D (use of PPh₃ as the ligand), the yield of 3-(p-tolyl)pyridine, as calculated by gas chromatography, is 93%. The yield of 3-(p-tolyl)pyridine, as calculated by isolation, is 77%.

In view of the foregoing, it has unexpectedly been found that a first aromatic compound having a fluorosulfonate substituent may be coupled to a second aromatic compound having a boron-containing substituent. Further, it has been found that the foregoing reaction can be performed in the presence of water. Further still, it is expected that the foregoing reactions require a lower quantity of catalysts as compared to similar reactions using triflates. Further, it is expected that the foregoing reactions require a lower quantity of ligands as compared to similar reactions using triflates. Additionally, it has been found that the foregoing reactions will not require a dedicated purification step, as is often necessary with similar reactions using triflates.

### Example 7. One pot conversion of 4-methylphenol to p-tolyl sulfofluoridate in the presence of an inorganic base followed by reaction of p-tolyl sulfofluoridate with phenylboronic acid to yield 4-phenyltoluene as shown in Equation 11.

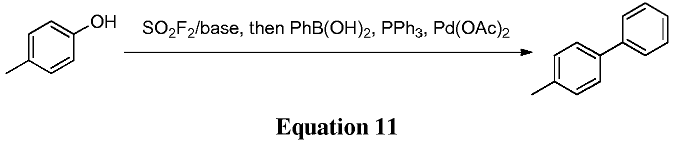

To two 30 mL vials is added 4-methylphenol (0.270 g; 2.50 mmol). To vial A is added potassium carbonate (1.20 g; 8.75 mmol). To vial B is added potassium phosphate (1.85 g; 8.75 mmol). To each vial is added SO₂F₂ in 1,4-dioxane (13.5 mL) via syringe. The vials are tightly capped and stirred at room temperature for 24 hours. The progress of each reaction is checked by gas chromatography / mass spectroscopy analysis which indicates greater than 95% conversion to p-tolyl sulfofluoridate.

The reaction mixtures are then degassed by bubbling N₂ gas for 15 minutes. The vials are taken into a N₂ filled glovebox, and in the following order water (1.50 mL), phenylboronic acid (0.427 g; 3.50 mmol), triphenylphosphine (0.0164 g) and Pd(OAc)₂ (0.0056 g) are added to the vials. The reaction mixtures are heated at 60 °C for 12 hours. The reaction mixtures are cooled to room temperature and each reaction is analyzed by gas chromatography / mass spectroscopy analysis which indicates that reaction A has undergone complete conversion to 4-phenyltoluene and reaction B has undergone >70% conversion to 4-phenyltoluene.

To vials A and B is then added 10 mL ethyl acetate and HCl (10% w/v). The organic layer is isolated and the aqueous layer is washed with ethyl acetate (20 mL). The combined organic fractions are absorbed onto silica gel and the product is purified by flash chromatography (-hexane/ethyl acetate gradient). Vial A: 4-phenyltoluene is isolated as a white solid (0.379 g; 90%) as verified by ¹H NMR. Vial B: 4-phenyltoluene is isolated as a white solid (0.270 g; 64%) as verified by ¹H NMR.

### Example 8. p-tolyl sulfofluoridate, phenylboronic acid reacted to yield 4-phenyltoluene

In this Example, p-tolyl sulfofluoridate is reacted with phenylboronic acid to yield 4-phenyltoluene as shown in Equation 12.

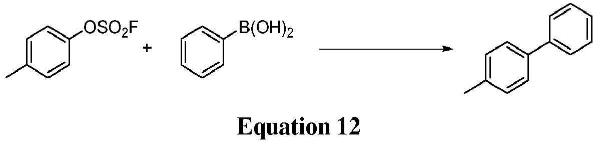

In a N₂ filled glovebox, 33 4 mL scintillation vials are each charged with (0.043 mL, 0.3 mmol, 1 equivalent) of p-tolyl sulfofluoridate and (0.24 mL of 1.5 molar solution of PBA in dioxane, 2 equivalents) of phenylboronic acid PBA. To each vial is also added the precatalyst (1-5 mol%) identified in Table 7. To some of the vials is added an additive (2-200 mol%) as identified in Table 7. As used herein, "additive" may refer to a ligand or to another compound, such as those identified in Table 7. To each vial is added (0.9 mmol, 3 equivalents) base identified in Table 7 and 2 mL of dioxane. The reaction mixture is stirred for 15 hours at 80°C. GC yields are measured by using 1,3,5-trimethoxybanzene as standard and reported in Table 7.

**Table 7**

| Vial | Precatalyst | Precatalys t(mol%) | Additive | Additiv e (mol%) | Base | GC yield (%) |
|---|---|---|---|---|---|---|
| 1 | Bis(tricyclohexylphosp hine)nickel(II) dichloride | 5 | PCy3 | 10 | K₃PO₄ | 59 |
| 2 | Bis(triphenylphosphine )nickel(II) dichloride | 5 | triphenylphosphine | 10 | K₃PO₄ | 5 |
| 3 | [1,2-Bis(diphenylphosphino )ethane]dichloronickel( II) | 5 | 1,2-Bis(diphenylphosp hino)ethane (dppe) | 5 | K₃PO₄ | 54 |
| 4 | [1,3-Bis(diphenylphosphino )propane]dichloronicke l(II) | 5 | | | K₃PO₄ | 24 |
| 5 | [1,1'-Bis(diphenylphosphino )ferrocene]dichloronick el(II) | 5 | | | K₃PO₄ | 4 |
| 6 | Nickel(II) chloride ethylene glycol dimethyl ether complex | 5 | PCy3 | 10 | K₃PO₄ | 23 |
| 7 | Bis(1,5-cyclooctadiene)nickel( 0) | 5 | PCy3 | 10 | K₃PO₄ | 22 |
| 8 | Bis(1,5-cyclooctadiene)nickel(0) | 5 | triphenylphosphine | 10 | K₃PO₄ | 7 |
| 9 | Bis(1,5-cyclooctadiene)nickel( 0) | 5 | PCy3 • HBF4 | 10 | K₃PO₄ | 66 |
| 10 | Bis(1,5-cyclooctadiene)nickel( 0) | 5 | 1,3-Bis(2,6-diisopropylphenyl) imidazolium chloride | 10 | K₃PO₄ | 16 |
| 11 | Bis(tricyclohexylphosp hine)nickel(II) dichloride | 5 | | | K₃PO₄ | 57 |
| 12 | Bis(triphenylphosphine )nickel(II) dichloride | 5 | | | K₃PO₄ | 3 |
| 13 | [1,2-Bis(diphenylphosphino )ethane]dichloronickel( II) | 5 | | | K₃PO₄ | 53 |
| 14 | [1,3-Bis(diphenylphosphino )propane]dichloronicke l(II) | 5 | Zn | 200 | K₃PO₄ | 14 |
| 15 | [1,1'-Bis(diphenylphosphino )ferrocene]dichloronick el(II) | 5 | Zn | 200 | K₃PO₄ | 10 |
| 16 | Bis(tricyclohexylphosp hine)nickel(II) dichloride | 5 | Zn | 200 | K₃PO₄ | 60 |
| 17 | Bis(triphenylphosphine )nickel(II) dichloride | 5 | Zn | 200 | K₃PO₄ | 6 |
| 18 | Bis(tricyclohexylphosp hine)nickel(II) dichloride | 1 | PCy3 | 2 | K₃PO₄ | 44 |
| 19 | Bis(tricyclohexylphosp hine)nickel(II) dichloride | 3 | PCy3 • HBF4 | 6 | K₃PO₄ | 91 |
| 20 | Bis(tricyclohexylphosp hine)nickel(II) dichloride | 1 | PCy3 • HBF4 | 2 | K₃PO₄ | 96 |
| 21 | Bis(1,5-cyclooctadiene)nickel( 0) | 3 | PCy3 | 6 | K₃PO₄ | 99 |
| 22 | Bis(1,5-cyclooctadiene)nickel( 0) | 3 | PCy3 • HBF4 | 6 | K₃PO₄ | 99 |
| 23 | Bis(1,5-cyclooctadiene)nickel( 0) | 1 | PCy3 | 3 | K₃PO₄ | 104 |
| 24 | Bis(1,5-cyclooctadiene)nickel( 0) | 1 | PCy3 • HBF4 | 3 | K₃PO₄ | 102 |
| 25 | Ni(OAc)2•4H2O | 5 | PCy3 | 15 | K₃PO₄ | 41 |
| 26 | Ni(OAc)2•4H2O | 5 | PCy3 • HBF4 | 15 | K₃PO₄ | 79 |
| 27 | Bis(tricyclohexylphosp hine)nickel(II) dichloride | 5 | | | Na₂CO₃ | 60 |
| 28 | Bis(1,5-cyclooctadiene)nickel( 0) | 5 | PCy3 • HBF4 | 10 | Na₂CO₃ | 72 |
| 29 | Bis(1,5-cyclooctadiene)nickel( 0) | 5 | PCy3 • HBF4 | 10 | Et₃N | 40 |
| 30 | Bis(1,5-cyclooctadiene)nickel( 0) | 5 | PCy3 | 10 | Et₃N | 40 |
| 31 | Bis(tricyclohexylphosp hine)nickel(II) dichloride | 5 | | | NaOtBu | 27 |
| 32 | Bis(1,5-cyclooctadiene)nickel( 0) | 5 | PCy3 • HBF4 | 10 | NaOtBu | 23 |
| 33 | Bis(1,5-cyclooctadiene)nickel( 0) | 5 | PCy3 | 10 | NaOtBu | 17 |

### Example 9. Substituted p-tolyl sulfofluoridate, phenylboronic acid reacted to yield substituted 4-phenyltoluene

In this Example, substituted p-tolyl sulfofluoridate is reacted with phenylboronic acid to yield substituted 4-phenyltoluene as shown in Equation 13. In Equation 13, -FG is a generic identifier that designates a functional group which is bonded to the ring at a desired position.

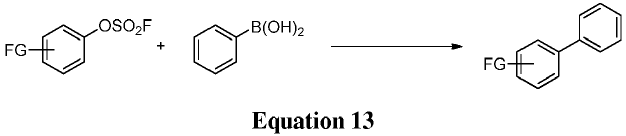

In a N₂ filled glovebox, eleven 4 mL scintillation vials are charged with (0.5 mmol, 1 equivalent) of the functionalized fluorosulfonate identified in Table 8. (1 mmol, 2 equivalents) of phenylboronic acid PBA are added to each vial as 1.5 molar solution in dioxane. To each vial is added NiCl₂(PCy₃)₂precatalyst (3.45 mg, 1 mol%) , PCy3 • HBF₄ (3.68 mg, 2 mol%), K₃PO₄ (0.318 g, 1.5 mmol, 3 equivalents) and 1,4-dioxane to have 2mL altogether volume. The reaction mixture is stirred for 15 hours at 80 °C. The reaction mixture is impregnated on silica gel and the product identified in Table 8 is the isolated yield is calculated using column chromatography and recorded in Table 8.

**Table 8**

| Vial | Fluorosulfonate | Product | Yield, mg | Isolated yield, % |
|---|---|---|---|---|
| 1 | 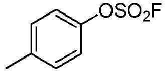 | 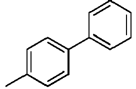 | 74 | 88 |
| 2 | 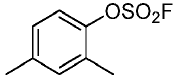 | 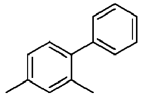 | 83 | 91 |
| 3 | 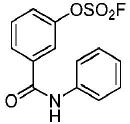 | 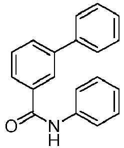 | 89 | 65 |
| 4 | 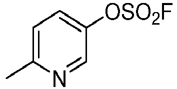 | 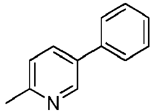 | 20 | 24 |
| 5 | 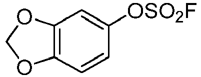 | 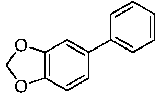 | 89 | 90 |
| 6 | 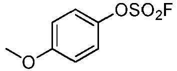 | 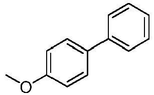 | 77 | 84 |
| 7 | 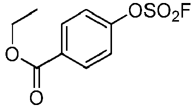 | 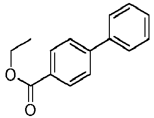 | 104 | 92 |
| 8 | 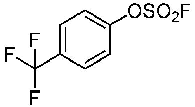 | 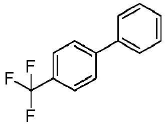 | 44 | 40 |
| 9 | 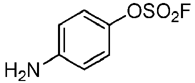 | 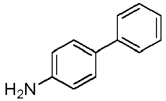 | 45 | 53 |
| 10 | 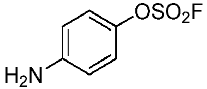 | 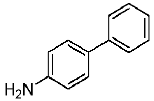 | 64 | 76 |
| 11 | 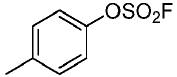 | 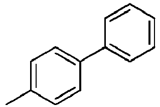 | 70 | 83 |

For vial 10 of the present example, the example is conducted as described herein except that the quantities of compounds are modified as follows: 0.5 mol % of NiCl₂(PCy₃) and 1 mol % of PCy3 • HBF₄. For vial 11 of the present example, the example is conducted as described herein except that the quantities of compounds are modified as follows: 2 mol % of PCy3 as an additive instead of 2 mol % of PCy₃•HBF₄.

### Example 10. 1-(4-hydroxyphenyl)ethanone, phenylboronic acid reacted to yield 1-([1,1'-biphenyl]-4-yl)ethanone.

In this Example, substituted 1-(4-hydroxyphenyl)ethanone is first treated with sulfurylfluoride and is second reacted with phenylboronic acid in a one-pot reaction scheme to yield 1-([1,1'-biphenyl]-4-yl)ethanoneas shown in Equation 14.

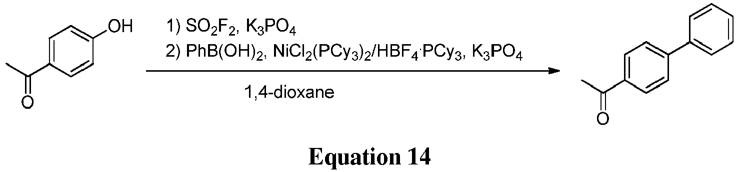

To a 20 mL vial is added 1-(4-hydroxyphenyl)ethanone (2.50 mmol), K₃PO₄ (8.75 mmol) followed by the addition of 3 wt% solution of SO₂F₂ (1.5 equiv.; 3.75 mmol) in dioxane. The reaction mixture is stirred at room temperature for 48 hours. Excess sulfurylfluoride is removed from the reaction mixture by purging with nitrogen (1 hour). To the reaction mixture are added 2 equivalents of phenylboronic acid PBA, 1.0 mol% of precatalyst - NiCl₂(PCy₃)₂, 2.0 mol% of PCy₃•HBF₄. The reaction mixture is stirred for 15 hours at 80 °C. The reaction mixture is impregnated on silica gel and the desired product is isolated with 67 % yield as measured using column chromatography (hexane/ethylacetate as eluent).

### Example 11. 4-hydroxybenzaldehyde, phenylboronic acid reacted to yield [1,1'-biphenyl]-4-carbaldehyde.

In this Example, 4-hydroxybenzaldehyde is first treated with sulfurylfluoride and is second reacted with phenylboronic acid in a one-pot reaction scheme to yield [1,1'-biphenyl]-4-carbaldehyde as shown in Equation 15.

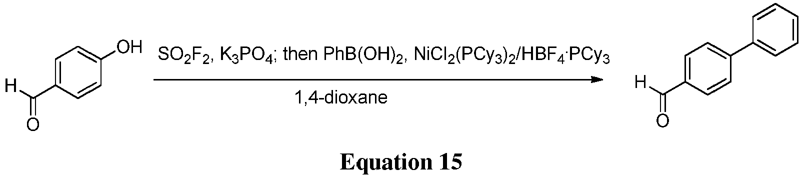

To a 20 mL vial is added 4-hydroxybenzaldehyde (2.50 mmol), K₃PO₄ (8.75 mmol) followed by the addition of 3 wt% solution of SO₂F₂ (1.5 equiv.; 3.75 mmol) in dioxane. The reaction mixture is stirred at RT for 48 hours. Excess of SO₂F₂ is removed by purging with nitrogen (1 hour). To the reaction mixture are added 2 equivalents of phenylboronic acid PBA, 1.0 mol% of precatalyst - NiCl₂(PCy₃)₂, 2.0 mol% of PCy₃•HBF₄. The reaction mixture is stirred for 15 hours at 80 °C. The reaction mixture is impregnated on silica gel and the desired product was isolated with 40 % yield as measured using column chromatography (hexane/ethylacetate as eluent).

### Example 12. 4-methylphenol, phenylboronic acid reacted to yield 4-methyl-1,1'-biphenyl.

In this Example, 4-methylphenol is first treated with sulfurylfluoride and is second reacted with phenylboronic acid in a one-pot reaction scheme to yield 4-methyl-1,1'-biphenyl as shown in Equation 16.

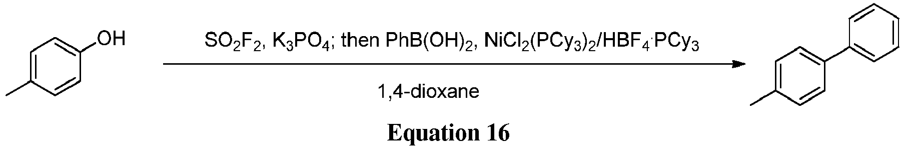

To a 20 mL vial is added 4-methylphenol (2.50 mmol), K₃PO₄ (8.75 mmol) followed by the addition of 3 wt% solution of SO₂F₂ (1.5 equiv.; 3.75 mmol) in dioxane. The reaction mixture is stirred at room temperature for 48 hours. Excess SO₂F₂ is removed from the vial by purging with nitrogen (1 hour). To the reaction mixture are added 2 equivalents of phenylboronic acid PBA, 1.0 mol% of precatalyst - NiCl₂(PCy₃)₂, and 2.0 mol% of PCy₃•HBF₄. The reaction mixture is stirred for 15 hours at 80 °C. Gas chromatograph yield is measured as 60% using 1,3,5-trimethoxybenzene as standard.

### Example 13. Substituted p-tolyl sulfofluoridate, phenylboronic acid reacted to yield substituted biaryl compound.

In this Example, substituted p-tolyl sulfofluoridate is reacted with phenylboronic acid to yield substituted biaryl compound as shown in Equation 13. In Equation 13, -FG is a generic identifier that designates a functional group which is bonded to the ring at a desired position.

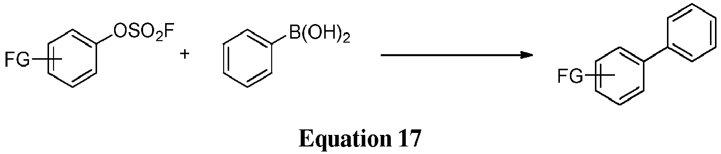

In a N₂ filled glovebox, thirteen 20 mL scintillation vial is charged with 2.25 mmol of the reactant identified in Table 9 (where R represents -SO₂F). 0.427 grams of phenylboronic acid are added to each vial. To each vial is added triphenylphosphine (0.49 ML of 0.127 M 1,4-dioxane solution); 6.44 mL 1,4-dioxane, 1.50 mL water and Pd(OAc)₂ (0.56 mL of 0.045 M acetonitrile solution). The reaction mixture of each vial is stirred for 12 hours at 60 °C. The reaction mixture of each vial is cooled to room temperature and is impregnated on silica gel and the product identified in Table 9 is the isolated yield is calculated using column chromatography and recorded in Table 9.

**Table 9**

| Vial | Reactant | Product | Fluorosulfonate (Example 13) | | Phenol (Example 14) | |
|---|---|---|---|---|---|---|
| | | | Yield (mg) | Isolated yield (%) | Yield (mg) | Isolated yield (%) |
| 1 | 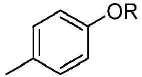 | 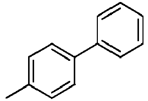 | 385 | 92 | 367 | 87 |
| 2 | 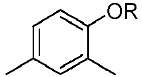 | 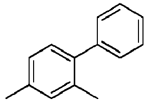 | 341 | 75 | 282 | 62 |
| 3 | 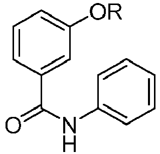 | 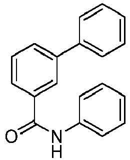 | 440 | 64 | 566 | 83 |
| 4 | 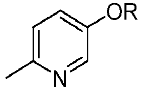 | 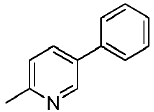 | 318 | 75 | 367 | 87 |
| 5 | 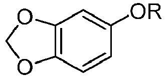 | 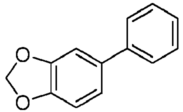 | 415 | 93 | 359 | 73 |
| 6 | 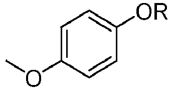 | 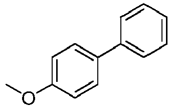 | 150 | 33 | 150 | 33 |
| 7 | 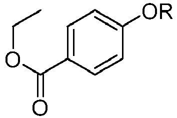 | 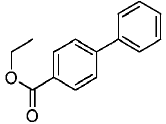 | 511 | 90 | 456 | 81 |
| 8 | 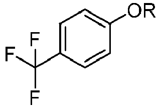 | 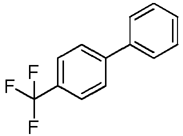 | 514 | 93 | 422 | 76 |
| 9 | 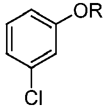 | 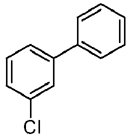 | 402 | 85 | 415 | 88 |
| 10 | 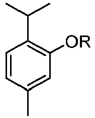 | 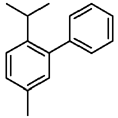 | 451 | 85 | 279 | 53 |
| 11 | 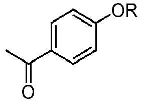 | 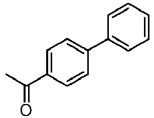 | 372 | 76 | - | - |
| 12 | 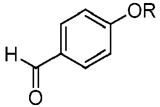 | 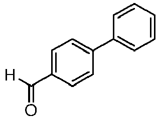 | 311 | 68 | - | - |
| 13 | 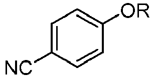 | 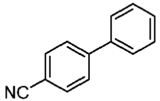 | 404 | 90 | - | - |

### Example 14. Substituted p-tolyl sulfofluoridate, phenylboronic acid reacted to yield substituted 4-phenyltoluene.

In this Example, substituted p-tolyl is first treated with sulfuryl fluoride and is second reacted with phenylboronic acid in a one-pot reaction scheme to yield the product identified in Table 9 as shown in Equation 18. In Equation 18, -FG is a generic identifier that designates a functional group which is bonded to the ring at a desired position.

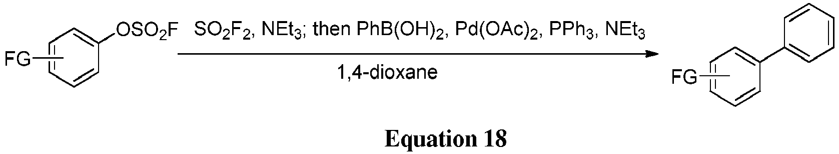

In a N₂ filled glovebox, 11 30 mL scintillation vial is charged with 2.50 mmol of the reactant identified in Table 9 (where R represents -H) and trimethylamine (1.2 mL; 8.75 mmol) (the reactions of vials 11-13 are not performed in this Example). To each vial is then added 13 mL of 3 weight percent solution of SO₂F₂ in 1.4-dioxane via syringe. Each vial is tightly capped and stirred at room temperature for 24 hours. The reaction mixture is degassed by bubbling nitrogen gas for 15 minutes. To each vial is added 0.427 grams of phenylboronic acid (3.50 mmol), 0.35 mL trimethylamine and 1.50 mL water. To each vial is added triphenylphosphine (0.50 ML of 0.125 M 1,4-dioxane solution); and Pd(OAc)₂ (0.50 mL of 0.05 M acetonitrile solution). The reaction mixture of each vial is stirred for 12 hours at 60 °C. The reaction mixture of each vial is cooled to room temperature and is impregnated on silica gel and the product identified in Table 9 is the isolated yield is calculated using column chromatography and recorded in Table 9.

### Example 15 p-tolyl sulfofluoridate, phenylboronic acid reacted to yield 4-phenyltoluene.

In this Example, p-tolyl sulfofluoridate is reacted with phenylboronic acid to yield 4-phenyltoluene as shown in Equation 19.

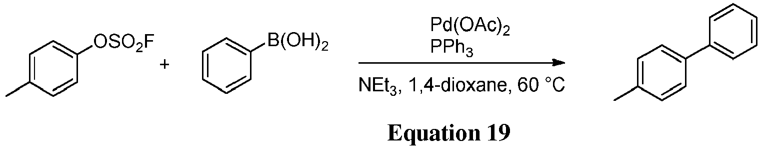

To four 20 mL vials is added p-tolyl sulfofluoridate (0.095 g; 0.50 mmol), phenyl boronic acid (0.085 g; 0.70 mmol), 1,4-dioxane (1.5 mL), water (0.3 mL), and triethylamine (0.19 mL). To each vial is added PPh₃ (0.076 M solution in acetonitrile) and Pd(OAc)₂ (0.045M solution in acetonitrile as described in the Table 10. The reaction mixture is heated at 60 °C for 24 hours and then cooled to room temperature. To each vial is added trimethoxybenzene (210 uL of 1.19M solution in 1,4-dioxane) as an internal standard. The reaction mixture is analyzed by a calibrated gas chromatographic method to determine the yield of 4-phenyltoluene in each reaction, as recorded in Table 10.

**Table 10**

| Vial | Catalyst loading (mol% Pd(OAc)₂ | Amount of Pd(OAc)₂ added | Amount of PPh₃ added | Yield of 4-phenyltoluene |
|---|---|---|---|---|
| 1 | 1.0 | 112 uL | 164 uL | 103% |
| 2 | 0.5 | 56 uL | 82 uL | 104% |
| 3 | 0.1 | 11 uL | 16 uL | 59% |
| 4 | 0.01 | 2.3 uL | 3.3 uL | 17% |

### Example 16 p-tolyl sulfofluoridate, phenylboronic acid pinacol ester reacted to yield 4-phenyltoluene.

In this Example, p-tolyl sulfofluoridate is reacted with phenylboronic acid pinacol ester to yield 4-phenyltoluene as shown in Equation 20.

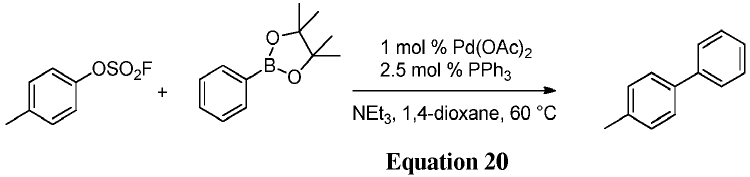

To a 20 mL vial is added p-tolyl sulfofluoridate (0.477 g; 2.50 mmol). phenylboronic acid pinacol ester (0.714g; 3.50 mmol), triethylamine (0.69 mL), PPh₃ (0.0164 g; 0.063 mmol),Pd(OAc)₂ (0.0056 g; 0.025 mmol), 1,4-dixoane (7.5mL) and water (1.5 mL). The reaction mixture is heated at 60 °C for 12 hours and then cooled to room temperature. To the reaction mixture is added trimethoxybenzene (630 uL of 1.19M solution in 1,4-dioxane) as an internal standard. The reaction mixture is analyzed by a calibrated gas chromatographic method to determine a 61% yield of 4-phenyltoluene.

### Example 17 p-tolyl sulfofluoridate, potassium phenyltrifluoroborate reacted to yield 4-phenyltoluene.

In this Example, p-tolyl sulfofluoridate is reacted with potassium phenyltrifluoroborate to yield 4-phenyltoluene as shown in Equation 21.

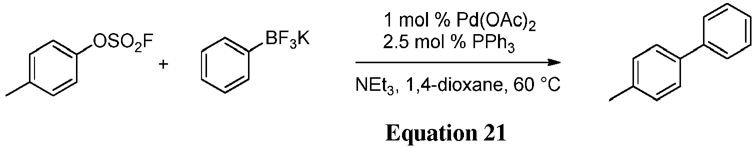

To a 20 mL vial is added p-tolyl sulfofluoridate (0.477 g; 2.50 mmol). Potassium phenyltrifluoroborate (0.644g; 3.50 mmol), triethylamine (0.69 mL), PPh₃ (0.0164 g; 0.063 mmol),Pd(OAc)₂ (0.0056 g; 0.025 mmol), 1,4-dixoane (7.5mL) and water (1.5 mL). The reaction mixture is heated at 60 °C for 12 hours and then cooled to room temperature. To the reaction mixture is added trimethoxybenzene (630 uL of 1.19M solution in 1,4-dioxane) as an internal standard. The reaction mixture is analyzed by a calibrated gas chromatographic method to determine a 33% yield of 4-phenyltoluene.

## Claims

1. A method of coupling a first aromatic compound to a second aromatic compound, the method comprising:
providing the first aromatic compound having a fluorosulfonate substituent;
providing the second aromatic compound having a boron-containing substituent; and
reacting the first aromatic compound and the second aromatic compound in a reaction mixture, the reaction mixture including a catalyst having at least one group 10 atom, the reaction mixture under conditions effective to couple the first aromatic compound to the second aromatic compound
wherein
- the first and second aromatic compounds are p-tolyl sulfofluoridate and phenylboronic acid respectively, which are reacted to yield 4-phenyltoluene, or
- the first and second aromatic compounds are p-tolyl sulfofluoridate and tolyl boronic acid respectively, which are reacted to yield 4,4'-dimethyl-1,1'-biphenyl, or
- the first and second aromatic compounds are 4-(trifluoromethyl)phenyl-sulfurofluoridate and phenyl boronic acid respectively, which are reacted to yield 4-trifluoromethyl-1,1'-biphenyl, or
- the first and second aromatic compounds are 4-(trifluoromethyl)phenyl-sulfurofluoridate and tolyl boronic acid respectively, which are reacted to yield 4-phenyltoluene, or
- the first and second aromatic compounds are pyridin-3-yl sulfurofluoridate and phenyl boronic acid respectively, which are reacted to yield 3-(p-tolyl)pyridine, or
- the first and second aromatic compounds are pyridin-3-yl sulfurofluoridate and p-tolyl boronic acid respectively, which are reacted to yield 3-(p-tolyl)pyridine, or
- the first and second aromatic compounds are substituted p-tolyl sulfofluoridateand phenylboronic acid respectively, which are reacted to yield substituted 4-phenyltoluene, wherein the substituted fluorosulfonate and the substituted 4-phenyltoluene are selected from the group consisting of the following substituted fluorosulfonate and substituted 4-phenyltoluenes:
| substituted fluorosulfonate | substituted 4-phenyltoluenes |
|---|---|
| 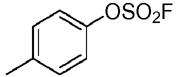 | 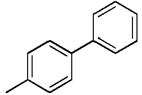 |
| 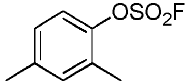 | 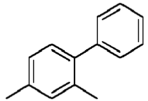 |
| 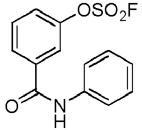 | 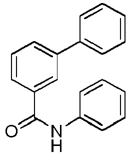 |
| 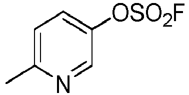 | 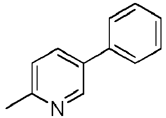 |
| 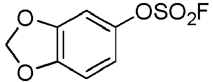 | 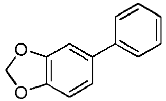 |
| 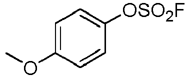 | 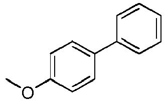 |
| 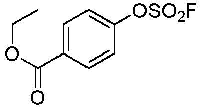 | 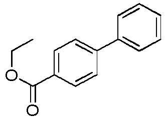 |
| 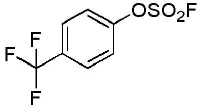 | 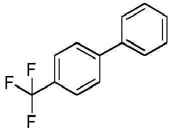 |
| 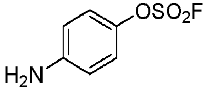 | 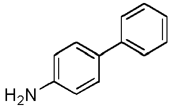 |
| 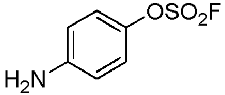 | 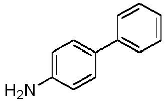 |
| 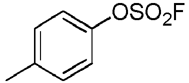 | 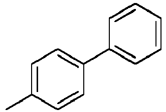 |
, or
- the first and second aromatic compounds are substituted p-tolyl sulfofluoridate and phenylboronic acid respectively, which are reacted to yield a substituted biaryl compound, wherein the substituted fluorosulfonate and the substituted biaryl compound are selected from the group consisting of the following substituted fluorosulfonates, and substituted biaryl compounds:
| substituted fluorosulfonates | substituted biaryl compounds |
|---|---|
| 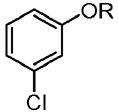 | 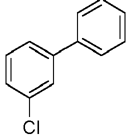 |
| 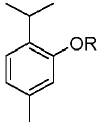 | 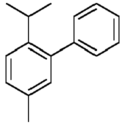 |
| 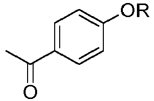 | 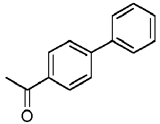 |
| 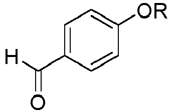 | 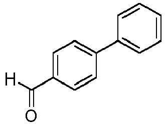 |
| 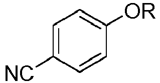 | 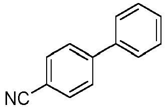 |
wherein R = SO₂F, or
- the first and second aromatic compounds are p-tolyl sulfofluoridate and phenylboronic acid pinacol ester respectively, which are reacted to yield 4-phenyltoluene, or
- the first and second aromatic compounds are p-tolyl sulfofluoridate and potassium phenyltrifluoroborate respectively, which are reacted to yield 4-phenyltoluene.

2. The method of claim 1, wherein the reaction mixture further includes a ligand, and a base.

3. The method of any one of claims 1-2, wherein at least one of the first aromatic compound or the second aromatic compound is heteroaryl.

4. The method of any one of claims 1-3 wherein the catalyst is a palladium catalyst or a nickel catalyst.

5. The method of claim 4, wherein the catalyst is generated in-situ from a palladium precatalyst, the palladium precatalyst is selected from the group consisting of: Palladium(II) acetate, Palladium(II) chloride, Dichlorobis(acetonitrile)palladium(II), Dichlorobis(benzonitrile)palladium(II), Allylpalladium chloride dimer, Palladium(II) acetylacetonate, Palladium(II) bromide, Bis(dibenzylideneacetone)palladium(0), Bis(2-methylallyl)palladium chloride dimer, Crotylpalladium chloride dimer, Dichloro(1,5-cyclooctadiene)palladium(II), Dichloro(norbornadiene)palladium(II), Palladium(II) trifluoroacetate, Palladium(II) benzoate, Palladium(II) trimethylacetate, Palladium(II) oxide, Palladium(II) cyanide, Tris(dibenzylideneacetone)dipalladium(0), Palladium(II) hexafluoroacetylacetonate, cis-Dichloro(N,N,N',N'-tetramethylethylenediamine)palladium(II), Cyclopentadienyl[(1,2,3-n)-1-phenyl-2-propenyl]palladium(II), [1,3-Bis(2,6-Diisopropylphenyl)imidazol-2-ylidene](3-chloropyridyl)palladium(II) dichloride, (1,3-Bis(2,6-diisopropylphenyl)imidazolidene)(3-chloropyridyl) palladium(II) dichloride, and a mixture of two or more thereof.

6. The method of claim 4, wherein the catalyst is generated in-situ from a nickel precatalyst, the nickel precatalyst is selected from the group consisting of: nickel(II) acetate, nickel(II) chloride, Bis(triphenylphosphine)nickel(II) dichloride, Bis(tricyclohexylphosphine) nickel(II) dichloride, [1,1'-Bis(diphenylphosphino)ferrocene]dichloronickel(II), Dichloro[1,2-bis(diethylphosphino)ethane]nickel(II), Chloro(1-naphthyl)bis(triphenylphosphine)nickel(II), 1,3-Bis(2,6-diisopropylphenyl)imidazolium chloride, Bis(1,5-cyclooctadiene)nickel(0), Nickel(II) chloride ethylene glycol dimethyl ether complex, [1,3-Bis(diphenylphosphino) propane]dichloronickel(II), [1,2-Bis(diphenylphosphino)ethane]dichloronickel(II), Bis(tricyclohexylphosphine)nickel(0).

7. The method of any one of claims 2-6 wherein the ligand includes one or more of a phosphine ligand, a carbene ligand, an amine-based ligand, an aminophosphine-based ligand.

8. The method of any one of claims 2-7, wherein the base is a carbonate salt, a phosphate salt, an acetate salt or a carboxylic acid salt.

9. The method of any one of claims 2-8, wherein the base is selected from the group consisting of lithium carbonate, sodium carbonate, potassium carbonate, rubidium carbonate, cesium carbonate, ammonium carbonate, substituted ammonium carbonates, hydrogen carbonates, lithium phosphate, sodium phosphate, potassium phosphate, rubidium phosphate, cesium phosphate, ammonium phosphate, substituted ammonium phosphates, hydrogen phosphates, lithium acetate, sodium acetate, potassium acetate, rubidium acetate, cesium acetate, ammonium acetate, substituted ammonium acetates, formate salts, fluoroacetate salts, propionate anions with lithium, sodium, potassium, rubidium, cesium, ammonium, and substituted ammonium cations, lithium hydroxide, sodium hydroxide, potassium hydroxide, magnesium dihydroxide, calcium dihydroxide, strontium dihydroxide, and barium dihydroxide, aluminum trihydroxide, gallium trihydroxide, indium trihydroxide, thallium trihydroxide, triethylamine, N,N-diisopropylethylamine, 1,4-diazabicyclo[2.2.2]octane, 1,5-Diazabicyclo[4.3.0]non-5-ene, 1,8-Diazabicyclo[5.4.0]undec-7-ene, lithium, sodium, and potassium salts of bis(trimethylsilyl)amide, lithium, sodium, and potassium salts of t butoxide, 1,8-bis(dimethylamino)naphthalene, pyridine, morpholine, 2,6-lutidine, triethylamine., N,N-Dicyclohexylmethylamine, diisopropylamine, sodium fluoride, potassium fluoride, cesium fluoride, silver fluoride, tetra butyl ammonium fluoride, ammonium fluoride, triethyl ammonium fluoride and a mixture of two or more thereof.

10. The method of any one of claims 1-9, wherein the reaction mixture includes a solvent, wherein the solvent is selected from the group consisting of toluene, xylenes (*ortho*-xylene, *meta*-xylene, *para-xylene* or mixtures thereof), benzene, water, methanol, ethanol, 1-propanol, 2-propanol, *n*-butanol, 2-butanol, pentanol, hexanol, *tert*-butyl alcohol, *tert*-amyl alcohol, ethylene glycol, 1,2-propanedioal, 1,3-propanediol, glycerol, *N*-methyl-2-pyrrolidone, acetonitrile, *N,N*-dimethylformamide, methyl acetate, ethyl acetate, propyl acetate, isopropyl acetate, triacetin, acetone, methyl ethyl ketone, and ethereal solvents, such as 1,4-dioxane, tetrahydrofuran, 2-methyltetrahydrofuran, diethylether, cyclopenyl methyl ether, 2-butyl ethyl ether, dimethoxyethane, and polyethyleneglycol.

11. The method of any one of claims 1-10, wherein the boron-containing substituent is of the formula -BF₃⁻M⁺ where M⁺ is an alkali metal cation or an unsubstituted ammonium ion.

12. The method of any one of claims 1-11, wherein the boron-containing substituent is of the form: 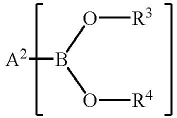 where R³ and R⁴ are each C₁₋₁₈ alkyl or C₆₋₁₈ aryl, H, B, or are covalently linked together to form a ring, and A² represents second aromatic compound.

13. A method of coupling a first aromatic compound to a second aromatic compound, the method comprising:
providing the first aromatic compound having a hydroxyl substituent;
providing sulfuryl fluoride in the presence of a base;
reacting the first aromatic compound and the sulfuryl fluoride in a reaction mixture, the reaction mixture under conditions effective to couple the sulfur atom of the sulfuryl fluoride to the oxygen of the hydroxyl group;
providing to the reaction mixture the second aromatic compound having a boron-containing substituent;
providing to the reaction mixture a catalyst having at least one group 10 atom; and
reacting the first aromatic compound and the second aromatic compound in the reaction mixture, the reaction mixture under conditions effective to couple the first aromatic compound to the second aromatic compound, and wherein the reaction is performed as a one-pot reaction.

14. The method of claim 13, the base comprising an inorganic base.

15. The method of claim 14, the base comprising an amine base.

16. The method of any one of claims 13-15, the catalyst comprising a nickel-based catalyst.

17. A method of coupling a first aromatic compound to a second aromatic compound, the method comprising:
providing the first aromatic compound having a fluorosulfonate substituent;
providing the second aromatic compound having a boron-containing substituent; and
reacting the first aromatic compound and the second aromatic compound in a reaction mixture, the reaction mixture including a catalyst having at least one group 10 atom, the reaction mixture under conditions effective to couple the first aromatic compound to the second aromatic compound
wherein the catalyst is a nickel-based catalyst.

## Patentansprüche

1. Ein Verfahren zum Verknüpfen einer ersten aromatischen Verbindung mit einer zweiten aromatischen Verbindung, wobei das Verfahren Folgendes beinhaltet:
Bereitstellen der ersten aromatischen Verbindung, die einen Fluorsulfonatsubstituenten aufweist;
Bereitstellen der zweiten aromatischen Verbindung, die einen borhaltigen Substituenten aufweist; und
Zur-Reaktion-Bringen der ersten aromatischen Verbindung und der zweiten aromatischen Verbindung in einer Reaktionsmischung, wobei die Reaktionsmischung einen Katalysator mit mindestens einem Atom der Gruppe 10 umfasst, wobei die Reaktionsmischung unter Bedingungen vorliegt, die wirksam sind, um die erste aromatische Verbindung mit der zweiten aromatischen Verbindung zu verknüpfen, wobei
- die erste und die zweite aromatische Verbindung p-Tolylsulfofluoridat bzw. Phenylboronsäure sind, die zur Reaktion gebracht werden, um 4-Phenyltoluol zu ergeben, oder
- die erste und die zweite aromatische Verbindung p-Tolylsulfofluoridat bzw. Tolylboronsäure sind, die zur Reaktion gebracht werden, um 4,4'-Dimethyl-1,1'-biphenyl zu ergeben, oder
- die erste und die zweite aromatische Verbindung 4-(Trifluormethyl)phenyl-sulfofluoridat bzw. Phenylboronsäure sind, die zur Reaktion gebracht werden, um 4-Trifluormethyl-1,1'-biphenyl zu ergeben, oder
- die erste und die zweite aromatische Verbindung 4-(Trifluormethyl)phenyl-sulfofluoridat bzw. Tolylboronsäure sind, die zur Reaktion gebracht werden, um 4-Phenyltoluol zu ergeben, oder
- die erste und die zweite aromatische Verbindung Pyridin-3-yl-sulfofluoridat bzw. Phenylboronsäure sind, die zur Reaktion gebracht werden, um 3-(p-Tolyl)pyridin zu ergeben, oder
- die erste und die zweite aromatische Verbindung Pyridin-3-yl-sulfofluoridat bzw. p-Tolylboronsäure sind, die zur Reaktion gebracht werden, um 3-(p-Tolyl)pyridin zu ergeben, oder
- die erste und die zweite aromatische Verbindung substituiertes p-Tolylsulfofluoridat bzw. Phenylboronsäure sind, die zur Reaktion gebracht werden, um substituiertes 4-Phenyltoluol zu ergeben, wobei das substituierte Fluorsulfonat und das substituierte 4-Phenyltoluol ausgewählt sind aus der Gruppe, bestehend aus dem nachfolgenden substituierten Fluorsulfonat und den nachfolgenden substituierten 4-Phenyltoluolen:
| substituiertes Fluorsulfonat | substituierte 4-Phenyltoluole |
|---|---|
| 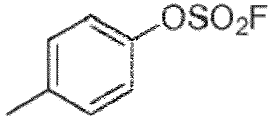 | 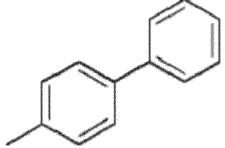 |
| 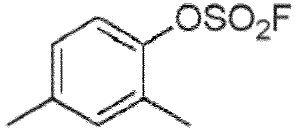 | 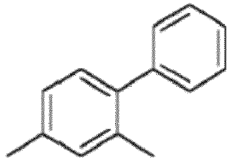 |
| 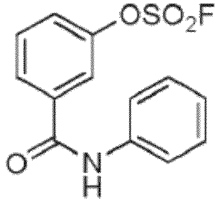 | 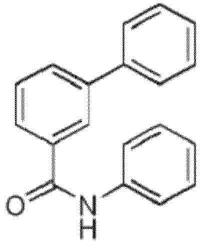 |
| 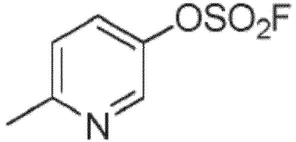 | 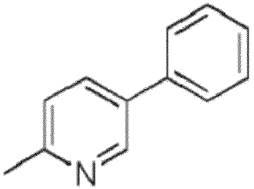 |
| 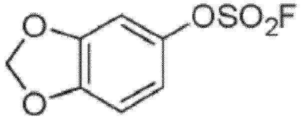 | 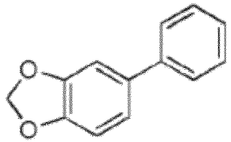 |
| 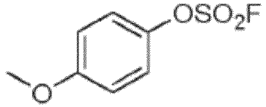 | 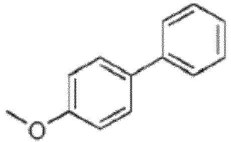 |
| 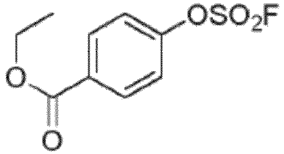 | 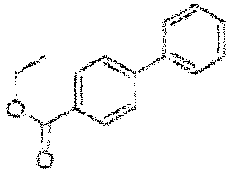 |
| 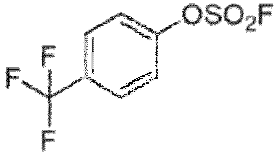 | 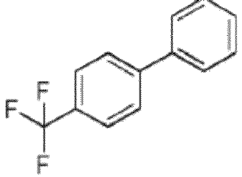 |
| 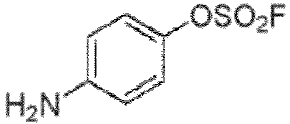 | 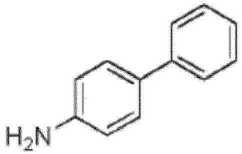 |
| 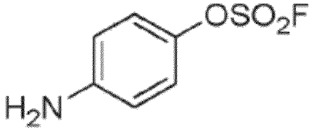 | 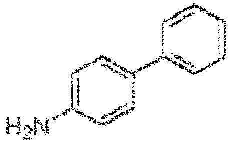 |
| 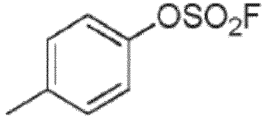 | 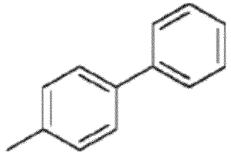 |
oder
- die erste und die zweite aromatische Verbindung substituiertes p-Tolylsulfofluoridat bzw. Phenylboronsäure sind, die zur Reaktion gebracht werden, um eine substituierte Biarylverbindung zu ergeben, wobei das substituierte Fluorsulfonat und die substituierte Biarylverbindung ausgewählt sind aus der Gruppe, bestehend aus den nachfolgenden substituierten Fluorsulfonaten und den nachfolgenden substituierten Biarylverbindungen:
| substituierte Fluorsulfonate | substituierte Biarylverbindungen |
|---|---|
| 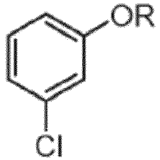 | 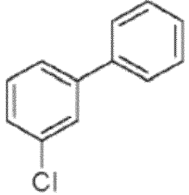 |
| 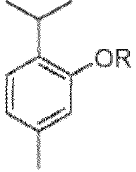 | 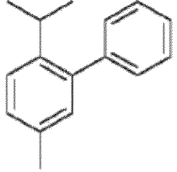 |
| 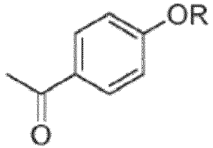 | 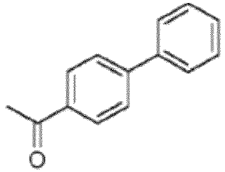 |
| 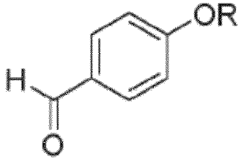 | 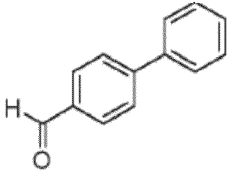 |
| 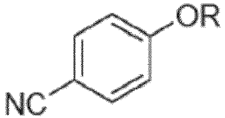 | 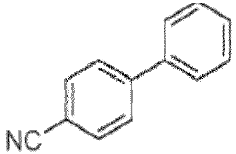 |
wobei R = SO₂F, oder
- die erste und die zweite aromatische Verbindung p-Tolylsulfofluoridat bzw. Phenylboronsäurepinacolester sind, die zur Reaktion gebracht werden, um 4-Phenyltoluol zu ergeben, oder
- die erste und die zweite aromatische Verbindung p-Tolylsulfofluoridat bzw. Kaliumphenyltrifluorborat sind, die zur Reaktion gebracht werden, um 4-Phenyltoluol zu ergeben.

2. Verfahren gemäß Anspruch 1, wobei die Reaktionsmischung ferner einen Liganden und eine Base umfasst.

3. Verfahren gemäß einem der Ansprüche 1-2, wobei mindestens eine von der ersten aromatischen Verbindung oder der zweiten aromatischen Verbindung Heteroaryl ist.

4. Verfahren gemäß einem der Ansprüche 1-3, wobei der Katalysator ein Palladiumkatalysator oder ein Nickelkatalysator ist.

5. Verfahren gemäß Anspruch 4, wobei der Katalysator in situ aus einem Palladium-Präkatalysator erzeugt wird, wobei der Palladium-Präkatalysator ausgewählt ist aus der Gruppe, bestehend aus Folgendem: Palladium(II)acetat, Palladium(II)chlorid, Dichlorbis(acetonitril)palladium(II), Dichlorbis(benzonitril)palladium(II), Allylpalladiumchloriddimer, Palladium(II)acetylacetonat, Palladium(II)bromid, Bis(dibenzylidenaceton)palladium(0), Bis(2-methylallyl)palladiumchloriddimer, Crotylpalladiumchloriddimer, Dichlor(1,5-cyclooctadien)palladium(II), Dichlor(norbornadien)palladium(II), Palladium(II)trifluoracetat, Palladium(II)benzoat, Palladium(II)trimethylacetat, Palladium(II)oxid, Palladium(II)cyanid, Tris(dibenzylidenaceton)dipalladium(0), Palladium(II)hexafluoracetylacetonat, cis-Dichlor(N,N,N',N'-tetramethylethylendiamin)palladium(II), Cyclopentadienyl[(1,2,3-n)-1-phenyl-2-propenyl]palladium(II), [1,3-Bis(2,6-diisopropylphenyl)imidazol-2-yliden](3-chlorpyridyl)palladium(II)dichlorid, (1,3-Bis(2,6-diisopropylphenyl)imidazoliden)(3-chlorpyridyl)palladium (II)dichlorid und eine Mischung von zwei oder mehreren davon.

6. Verfahren gemäß Anspruch 4, wobei der Katalysator in situ aus einem Nickel-Präkatalysator erzeugt wird, wobei der Nickel-Präkatalysator ausgewählt ist aus der Gruppe, bestehend aus Folgendem: Nickel(II)acetat, Nickel(II)chlorid, Bis(triphenylphosphin)nickel(II)dichlorid, Bis(tricyclohexylphosphin)nickel(II)dichlorid, [1,1'-Bis(diphenylphosphino)ferrocen]dichlornickel(II), Dichlor[1,2-bis(diethylphosphino)ethan]nickel(II), Chlor(1-naphthyl)bis(triphenylphosphin)nickel(II), 1,3-Bis(2,6-diisopropylphenyl)imidazoliumchlorid, Bis(1,5-cyclooctadien)nickel(0), Nickel(II)chlorid-Ethylenglycoldimethyletherkomplex, [1,3-Bis(diphenylphosphino)propan]dichlornickel(II), [1,2-Bis(diphenylphosphino)ethan]dichlornickel(II), Bis(tricyclohexylphosphin)nickel(0).

7. Verfahren gemäß einem der Ansprüche 2-6, wobei der Ligand einen oder mehrere von einem Phosphinliganden, einem Carbenliganden, einem Liganden auf Aminbasis, einem Liganden auf Aminophosphinbasis umfasst.

8. Verfahren gemäß einem der Ansprüche 2-7, wobei die Base ein Carbonatsalz, ein Phosphatsalz, ein Acetatsalz oder ein Carbonsäuresalz ist.

9. Verfahren gemäß einem der Ansprüche 2-8, wobei die Base ausgewählt ist aus der Gruppe, bestehend aus Lithiumcarbonat, Natriumcarbonat, Kaliumcarbonat, Rubidiumcarbonat, Cäsiumcarbonat, Ammoniumcarbonat, substituierten Ammoniumcarbonaten, Hydrogencarbonaten, Lithiumphosphat, Natriumphosphat, Kaliumphosphat, Rubidiumphosphat, Cäsiumphosphat, Ammoniumphosphat, substituierten Ammoniumphosphaten, Hydrogenphosphaten, Lithiumacetat, Natriumacetat, Kaliumacetat, Rubidiumacetat, Cäsiumacetat, Ammoniumacetat, substituierten Ammoniumacetaten, Formiatsalzen, Fluoracetatsalzen, Propionatanionen mit Lithium-, Natrium-, Kalium-, Rubidium-, Cäsium-, Ammonium- und substituierten Ammoniumkationen, Lithiumhydroxid, Natriumhydroxid, Kaliumhydroxid, Magnesiumdihydroxid, Calciumdihydroxid, Strontiumdihydroxid und Bariumdihydroxid, Aluminiumtrihydroxid, Galliumtrihydroxid, Indiumtrihydroxid, Thalliumtrihydroxid, Triethylamin, N,N-Diisopropylethylamin, 1,4-Diazabicyclo[2.2.2]octan, 1,5-Diazabicyclo[4.3.0]non-5-en, 1,8-Diazabicyclo[5.4.0]undec-7-en, Lithium-, Natrium- und Kaliumsalzen von Bis(trimethylsilyl)amid, Lithium-, Natrium- und Kaliumsalzen von tert-Butoxid, 1,8-Bis(dimethylamino)naphthalin, Pyridin, Morpholin, 2,6-Lutidin, Triethylamin, N,N-Dicyclohexylmethylamin, Diisopropylamin, Natriumfluorid, Kaliumfluorid, Cäsiumfluorid, Silberfluorid, Tetrabutylammoniumfluorid, Ammoniumfluorid, Triethylammoniumfluorid und einer Mischung aus zwei oder mehreren davon.

10. Verfahren gemäß einem der Ansprüche 1-9, wobei die Reaktionsmischung ein Lösungsmittel umfasst, wobei das Lösungsmittel ausgewählt ist aus der Gruppe, bestehend aus Toluol, Xylolen (*ortho*-Xylol, *meta*-Xylol, *para*-Xylol oder Mischungen davon), Benzol, Wasser, Methanol, Ethanol, 1-Propanol, 2-Propanol, *n*-Butanol, 2-Butanol, Pentanol, Hexanol, *tert*-Butylalkohol, *tert*-Amylalkohol, Ethylenglykol, 1,2-Propandiol, 1,3-Propandiol, Glycerin, *N*-Methyl-2-pyrrolidon, Acetonitril, *N*,*N-*Dimethylformamid, Methylacetat, Ethylacetat, Propylacetat, Isopropylacetat, Triacetin, Aceton, Methylethylketon und etherischen Lösungsmitteln, wie etwa 1,4-Dioxan, Tetrahydrofuran, 2-Methyltetrahydrofuran, Diethylether, Cyclopenylmethylether, 2-Butylethylether, Dimethoxyethan und Polyethylenglycol.

11. Verfahren gemäß einem der Ansprüche 1-10, wobei der borhaltige Substituent die Formel -BF₃⁻M⁺ aufweist, wobei M⁺ ein Alkalimetallkation oder ein unsubstituiertes Ammoniumion ist

12. Verfahren gemäß einem der Ansprüche 1-11, wobei der borhaltige Substituent die folgende Form aufweist: 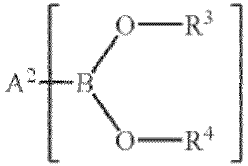 wobei R³ und R⁴ jeweils C₁₋₁₈-Alkyl oder C₆₋₁₈-Aryl, H, B sind oder kovalent miteinander verbunden sind, um einen Ring zu bilden, und A² für die zweite aromatische Verbindung steht.

13. Ein Verfahren zum Verknüpfen einer ersten aromatischen Verbindung mit einer zweiten aromatischen Verbindung, wobei das Verfahren Folgendes beinhaltet:
Bereitstellen der ersten aromatischen Verbindung, die einen Hydroxylsubstituenten aufweist;
Bereitstellen von Sulfurylfluorid in Gegenwart einer Base;
Zur-Reaktion-Bringen der ersten aromatischen Verbindung und desSulfurylfluorids in einer Reaktionsmischung, wobei die Reaktionsmischung unter Bedingungen vorliegt, die wirksam sind, um das Schwefelatom des Sulfurylfluorids mit dem Sauerstoff der Hydroxylgruppe zu verknüpfen;
Bereitstellen der zweiten, einen borhaltigen Substituenten aufweisenden, aromatischen Verbindung an die Reaktionsmischung;
Bereitstellen eines Katalysators, der mindestens ein Atom der Gruppe 10 aufweist, an die Reaktionsmischung; und Zur-Reaktion-Bringen der ersten aromatischen Verbindung und der zweiten aromatischen Verbindung in der Reaktionsmischung, wobei die Reaktionsmischung unter Bedingungen vorliegt, die wirksam sind, um die erste aromatische Verbindung mit der zweiten aromatischen Verbindung zu verknüpfen, und
wobei die Reaktion als eine Eintopfreaktion durchgeführt wird.

14. Verfahren gemäß Anspruch 13, wobei die Base eine anorganische Base beinhaltet.

15. Verfahren gemäß Anspruch 14, wobei die Base eine Aminbase beinhaltet.

16. Verfahren gemäß einem der Ansprüche 13-15, wobei der Katalysator einen Katalysator auf Nickelbasis beinhaltet.

17. Ein Verfahren zum Verknüpfen einer ersten aromatischen Verbindung mit einer zweiten aromatischen Verbindung, wobei das Verfahren Folgendes beinhaltet:
Bereitstellen der ersten aromatischen Verbindung, die einen Fluorsulfonatsubstituenten aufweist;
Bereitstellen der zweiten aromatischen Verbindung, die einen borhaltigen Substituenten aufweist; und
Zur-Reaktion-Bringen der ersten aromatischen Verbindung und der zweiten aromatischen Verbindung in einer Reaktionsmischung, wobei die Reaktionsmischung einen Katalysator mit mindestens einem Atom der Gruppe 10 umfasst, wobei die Reaktionsmischung unter Bedingungen vorliegt, die wirksam sind, um die erste aromatische Verbindung mit der zweiten aromatischen Verbindung zu verknüpfen, wobei der Katalysator ein Katalysator auf Nickelbasis ist.

## Revendications

1. Une méthode de couplage d'un premier composé aromatique à un deuxième composé aromatique, la méthode comprenant :
le fait de fournir le premier composé aromatique ayant un substituant fluorosulfonate ;
le fait de fournir le deuxième composé aromatique ayant un substituant contenant du bore ; et
le fait de faire réagir le premier composé aromatique et le deuxième composé aromatique dans un mélange réactionnel, le mélange réactionnel incluant un catalyseur ayant au moins un atome du groupe 10, le mélange réactionnel étant dans des conditions efficaces pour coupler le premier composé aromatique au deuxième composé aromatique
dans laquelle
- les premier et deuxième composés aromatiques sont le sulfofluoridate de p-tolyle et l'acide phénylboronique respectivement, lesquels sont amenés à réagir pour donner du 4-phényltoluène, ou
- les premier et deuxième composés aromatiques sont le sulfofluoridate de p-tolyle et l'acide tolylboronique respectivement, lesquels sont amenés à réagir pour donner du 4,4'-diméthyl-1,1'-biphényle, ou
- les premier et deuxième composés aromatiques sont le sulfurofluoridate de 4-(trifluorométhyl)phényle et l'acide phénylboronique respectivement, lesquels sont amenés à réagir pour donner du 4-trifluorométhyl-1,1'-biphényle, ou
- les premier et deuxième composés aromatiques sont le sulfurofluoridate de 4-(trifluorométhyl)phényle et l'acide tolylboronique respectivement, lesquels sont amenés à réagir pour donner du 4-phényltoluène, ou
- les premier et deuxième composés aromatiques sont le sulfurofluoridate de pyridin-3-yle et l'acide phénylboronique respectivement, lesquels sont amenés à réagir pour donner de la 3-(p-tolyl)pyridine, ou
- les premier et deuxième composés aromatiques sont le sulfurofluoridate de pyridin-3-yle et l'acide p-tolylboronique respectivement, lesquels sont amenés à réagir pour donner de la 3-(p-tolyl)pyridine, ou
- les premier et deuxième composés aromatiques sont le sulfofluoridate de p-tolyle substitué et l'acide phénylboronique respectivement, lesquels sont amenés à réagir pour donner du 4-phényltoluène substitué, le fluorosulfonate substitué et le 4-phényltoluène substitué étant sélectionnés dans le groupe constitué du fluorosulfonate substitué et des 4-phényltoluènes substitués suivants :
| fluorosulfonate substitué | 4-phényltoluènes substitués |
|---|---|
| 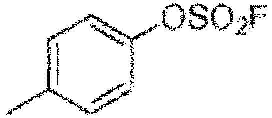 | 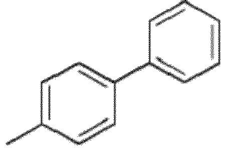 |
| 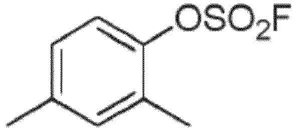 | 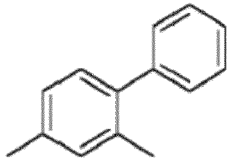 |
| 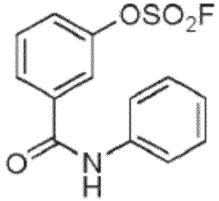 | 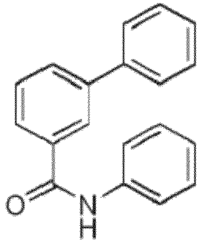 |
| 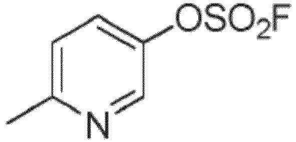 | 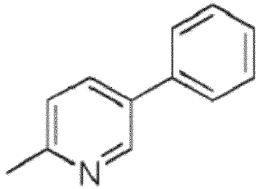 |
| 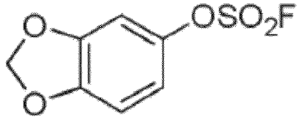 | 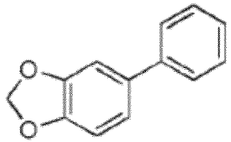 |
| 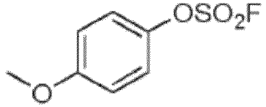 | 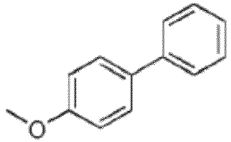 |
| 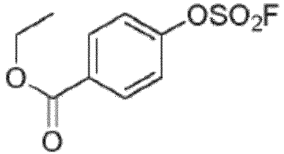 | 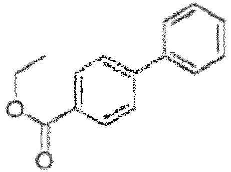 |
| 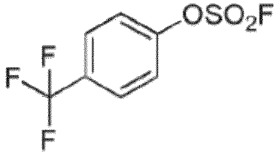 | 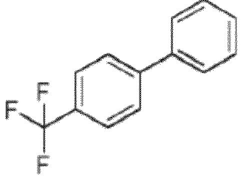 |
| 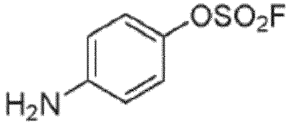 | 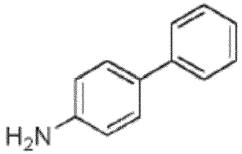 |
| 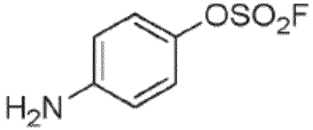 | 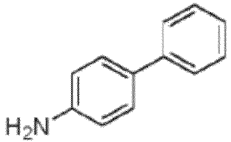 |
| 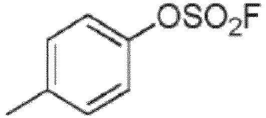 | 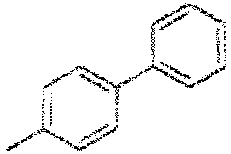 |
, ou
- les premier et deuxième composés aromatiques sont le sulfofluoridate de p-tolyle substitué et l'acide phénylboronique respectivement, lesquels sont amenés à réagir pour donner un composé biaryle substitué, le fluorosulfonate substitué et le composé biaryle substitué étant sélectionnés dans le groupe constitué des fluorosulfonates substitués et des composés biaryle substitués suivants :
| fluorosulfonates substitués | composés biaryle substitués |
|---|---|
| 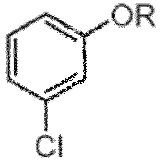 | 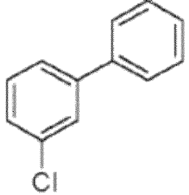 |
| 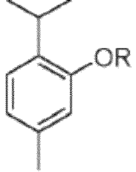 | 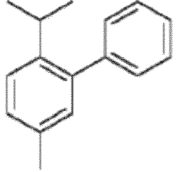 |
| 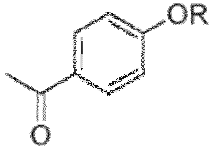 | 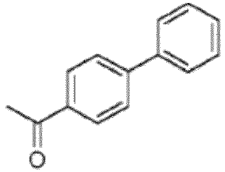 |
| 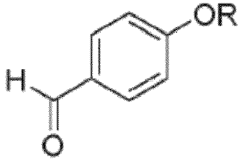 | 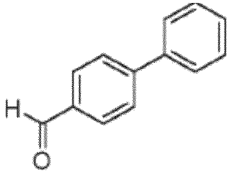 |
| 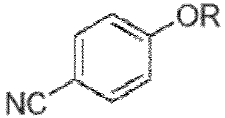 | 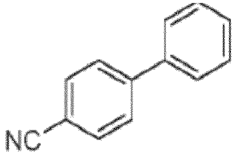 |
dans lesquels R = SO₂F, ou
- les premier et deuxième composés aromatiques sont le sulfofluoridate de p-tolyle et l'ester pinacolique d'acide phénylboronique respectivement, lesquels sont amenés à réagir pour donner du 4-phényltoluène, ou
- les premier et deuxième composés aromatiques sont le sulfofluoridate de p-tolyle et le phényltrifluoroborate de potassium respectivement, lesquels sont amenés à réagir pour donner du 4-phényltoluène.

2. La méthode de la revendication 1, dans laquelle le mélange réactionnel inclut en outre un ligand, et une base.

3. La méthode de n'importe laquelle des revendications 1 à 2, dans laquelle soit le premier composé aromatique, soit le deuxième composé aromatique est un hétéroaryle.

4. La méthode de n'importe laquelle des revendications 1 à 3 dans laquelle le catalyseur est un catalyseur au palladium ou un catalyseur au nickel.

5. La méthode de la revendication 4, dans laquelle le catalyseur est engendré in-situ à partir d'un précatalyseur au palladium, le précatalyseur au palladium est sélectionné dans le groupe constitué : de l'acétate de Palladium(II), du chlorure de Palladium(II), du Dichlorobis(acétonitrile)palladium(II), du Dichlorobis(benzonitrile)palladium(II), d'un dimère de chlorure d'Allylpalladium, de l'acétylacétonate de Palladium(II), du bromure de Palladium(II), du Bis(dibenzylidèneacétone)palladium(0), d'un dimère de chlorure de Bis(2-méthylallyl)palladium, d'une dimère de chlorure de Crotylpalladium, du Dichloro(1,5-cyclooctadiène)palladium(II), du Dichloro(norbornadiène)palladium(II), du trifluoroacétate de Palladium(II), du benzoate de Palladium(II), du triméthylacétate de Palladium(II), de l'oxyde de Palladium(II), du cyanure de Palladium(II), du Tris(dibenzylidèneacétone)dipalladium(0), de l'hexafluoroacétylacétonate de Palladium(II), du cis-Dichloro(N,N,N',N'-tétraméthyléthylène diamine)palladium(II), du Cyclopentadiényl[(1,2,3-n)-1-phényl-2-propényl]palladium(II), du dichlorure de [1,3-Bis(2,6-Diisopropylphényl)imidazol-2-ylidène](3-chloropyridyl)palladium(II), du dichlorure de (1,3-Bis(2,6-diisopropylphényl)imidazolidène)(3-chloropyridyl)palladium(II), et d'un mélange de deux de ceux-ci ou plus.

6. La méthode de la revendication 4, dans laquelle le catalyseur est engendré in-situ à partir d'un précatalyseur au nickel, le précatalyseur au nickel est sélectionné dans le groupe constitué : de l'acétate de nickel(II), du chlorure de nickel(II), du dichlorure de Bis(triphénylphosphine)nickel(II), du dichlorure de Bis(tricyclohexylphosphine)nickel(II), du [1,1'-Bis(diphénylphosphino)ferrocène]dichloronickel(II), du Dichloro[1,2-bis(diéthylphosphino)éthane]nickel(II), du Chloro(1-naphtyl)bis(triphénylphosphine)nickel(II), du chlorure de 1,3-Bis(2,6-diisopropylphényl)imidazolium, du Bis(1,5-cyclooctadiène)nickel(0), d'un complexe d'éther diméthylique d'éthylène glycol et de chlorure de Nickel(II), du [1,3-Bis(diphénylphosphino)propane]dichloronickel(II), du [1,2-Bis(diphénylphosphino)éthane]dichloronickel(II), du Bis(tricyclohexylphosphine)nickel(0).

7. La méthode de n'importe laquelle des revendications 2 à 6 dans laquelle le ligand inclut un ou plusieurs éléments parmi un ligand phosphine, un ligand carbène, un ligand à base d'amine, un ligand à base d'aminophosphine.

8. La méthode de n'importe laquelle des revendications 2 à 7, dans laquelle la base est un sel de carbonate, un sel de phosphate, un sel d'acétate ou un sel d'acide carboxylique.

9. La méthode de n'importe laquelle des revendications 2 à 8, dans laquelle la base est sélectionnée dans le groupe constitué du carbonate de lithium, du carbonate de sodium, du carbonate de potassium, du carbonate de rubidium, du carbonate de césium, du carbonate d'ammonium, de carbonates d'ammonium substitués, de carbonates d'hydrogène, du phosphate de lithium, du phosphate de sodium, du phosphate de potassium, du phosphate de rubidium, du phosphate de césium, du phosphate d'ammonium, de phosphates d'ammonium substitués, de phosphates d'hydrogène, de l'acétate de lithium, de l'acétate de sodium, de l'acétate de potassium, de l'acétate de rubidium, de l'acétate de césium, de l'acétate d'ammonium, d'acétates d'ammonium substitués, de sels de formiate, de sels de fluoroacétate, d'anions propionate avec du lithium, du sodium, du potassium, du rubidium, du césium, de l'ammonium, et de cations ammonium substitués, de l'hydroxyde de lithium, de l'hydroxyde de sodium, de l'hydroxyde de potassium, du dihydroxyde de magnésium, du dihydroxyde de calcium, du dihydroxyde de strontium, et du dihydroxyde de baryum, du trihydroxyde d'aluminium, du trihydroxyde de gallium, du trihydroxyde d'indium, du trihydroxyde de thallium, de la triéthylamine, de la N,N-diisopropyléthylamine, du 1,4-diazabicyclo[2.2.2]octane, du 1,5-Diazabicyclo[4.3.0] non-5-ène, du 1,8-Diazabicyclo[5.4.0]undéc-7-ène, de sels de lithium, de sodium, et de potassium de bis(triméthylsilyl)amide, de sels de lithium, de sodium, et de potassium de t-butoxyde, du 1,8-bis(diméthylamino)naphtalène, de la pyridine, de la morpholine, de la 2,6-lutidine, de la triéthylamine, de la N,N-Dicyclohexylméthylamine, de la diisopropylamine, du fluorure de sodium, du fluorure de potassium, du fluorure de césium, du fluorure d'argent, du fluorure de tétrabutylammonium, du fluorure d'ammonium, du fluorure de triéthylammonium et d'un mélange de deux de ceux-ci ou plus.

10. La méthode de n'importe laquelle des revendications 1 à 9, dans laquelle le mélange réactionnel inclut un solvant, le solvant étant sélectionné dans le groupe constitué du toluène, de xylènes (*ortho*-xylène, *méta*-xylène, para-xylène ou mélanges de ceux-ci), du benzène, de l'eau, du méthanol, de l'éthanol, du 1-propanol, du 2-propanol, du *n-*butanol, du 2-butanol, du pentanol, de l'hexanol, de l'alcool *tert-*butylique, de l'alcool *tert*-amylique, de l'éthylène glycol, du 1,2-propanediol, du 1,3-propanediol, du glycérol, de la *N*-méthyl-2- pyrrolidone, de l'acétonitrile, du *N,N-*diméthylformamide, de l'acétate de méthyle, de l'acétate d'éthyle, de l'acétate de propyle, de l'acétate d'isopropyle, de la triacétine, de l'acétone, de la méthyléthyl cétone, et de solvants éthérés, tels que le 1,4-dioxane, le tétrahydrofurane, le 2-méthyltétrahydrofurane, l'éther diéthylique, l'éther cyclopénylméthylique, l'éther 2-butyléthylique, le diméthoxyéthane, et le polyéthylène glycol.

11. La méthode de n'importe laquelle des revendications 1 à 10, dans laquelle le substituant contenant du bore est de la formule -BF₃⁻M⁺ où M⁺ est un cation métallique alcalin ou un ion ammonium non substitué.

12. La méthode de n'importe laquelle des revendications 1 à 11, dans laquelle le substituant contenant du bore est sous la forme : 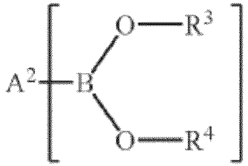 où R³ et R⁴ sont chacun un alkyle en C₁₋₁₈ ou un aryle en C₆₋₁₈, H, B, ou sont liés ensemble par covalence pour former un cycle, et A² représente un deuxième composé aromatique.

13. Une méthode de couplage d'un premier composé aromatique à un deuxième composé aromatique, la méthode comprenant :
le fait de fournir le premier composé aromatique ayant un substituant hydroxyle ;
le fait de fournir du fluorure de sulfuryle en présence d'une base ;
le fait de faire réagir le premier composé aromatique et le fluorure de sulfuryle dans un mélange réactionnel, le mélange réactionnel étant dans des conditions efficaces pour coupler l'atome de soufre du fluorure de sulfuryle à l'oxygène du groupe hydroxyle ;
le fait de fournir au mélange réactionnel le deuxième composé aromatique ayant un substituant contenant du bore ;
le fait de fournir au mélange réactionnel un catalyseur ayant au moins un atome du groupe 10 ; et
le fait de faire réagir le premier composé aromatique et le deuxième composé aromatique dans le mélange réactionnel, le mélange réactionnel étant dans des conditions efficaces pour coupler le premier composé aromatique au deuxième composé aromatique, et dans laquelle la réaction est effectuée en tant que réaction monotope.

14. La méthode de la revendication 13, la base comprenant une base inorganique.

15. La méthode de la revendication 14, la base comprenant une base amine.

16. La méthode de n'importe laquelle des revendications 13 à 15, le catalyseur comprenant un catalyseur à base de nickel.

17. Une méthode de couplage d'un premier composé aromatique à un deuxième composé aromatique, la méthode comprenant :
le fait de fournir le premier composé aromatique ayant un substituant fluorosulfonate ;
le fait de fournir le deuxième composé aromatique ayant un substituant contenant du bore ; et
le fait de faire réagir le premier composé aromatique et le deuxième composé aromatique dans un mélange réactionnel, le mélange réactionnel incluant un catalyseur ayant au moins un atome du groupe 10, le mélange réactionnel étant dans des conditions efficaces pour coupler le premier composé aromatique au deuxième composé aromatique
dans laquelle le catalyseur est un catalyseur à base de nickel.
